# EUROPEAN PATENT APPLICATION

(11) **EP 3 272 877 A1**
(43) Date of publication of application: **24.01.2018**
(21) Application number: 16180000.8
(22) Date of filing: 18.07.2016
(51) Int. Cl.: C12Q 1/68, A61K 48/00, C12N 15/63

(54) **B-CELL-MIMETIC CELLS**

(71) Applicant: ETH Zurich, 8092 Zurich (CH)
(72) Inventor: FUSSENEGGER, Martin, 5506 Mägenwil (CH); XIE, Mingqi, 4054 Basel (CH)

(57) **Abstract**

The present invention relates to β-cell-mimetic cells. Methods for producing β-cell-mimetic cells as well as methods of use of β-cell-mimetic cells as a medicament and methods of use of β-cell-mimetic cells for the prevention, delay of progression or treatment of a metabolic disease in a subject are also provided.

## Description

The present invention relates to β-cell-mimetic cells. Methods for producing β-cell-mimetic cells as well as methods of use of β-cell-mimetic cells as a medicament and methods of use of β-cell-mimetic cells for the prevention, delay of progression or treatment of a metabolic disease in a subject are also provided.

### BACKGROUND OF THE INVENTION

Diabetes mellitus is a complex and progressive disease with a pathophysiology involving metabolic impairments that can lead to many clinical complications. Diabetes mellitus is currently estimated to affect at least 415 million people (1 in 11 adults) worldwide (Diabetes Atlas 7Th Edition, International Diabetes Federation, 2015), a number already exceeding the value for 2025 predicted a decade ago. The most characteristic feature of diabetic patients is a chronically elevated blood-glucose level, known as hyperglycaemia, that results from either an absolute loss of pancreatic insulin-producing β-cells (type-1 diabetes, T1D) or a progressive exhaustion of active β-cells due to environmental factors such as a sedentary lifestyle, malnutrition, or obesity (type-2 diabetes, T2D). Unless sufficiently treated in time, sustained hyperglycaemia can initiate many pathologic cascades that result in more severe disorders such as cardiovascular disease, renal failure, the metabolic syndrome, neuropathic pain, hormone dysfunction and cancer. Therefore, improved glycaemic control by a therapeutic intervention that either enables tightly controlled insulin delivery or restores a patient's β-cell function will be of utmost importance in diabetes treatment.

Because T1D patients suffer from complete insulin deficiency due to a selective autoimmune destruction of β-cells treatment options focus on a disciplined or automated supply of exogenous insulin. By contrast, the number of possible drug targets for T2D therapy is higher due to the progressive and multifactorial nature of this disease type. For example, incretin hormones (e.g., GLP-1-analogues) improve the efficiency of the exhausting β-cells to secrete insulin upon glucose stimulation. In recent years, studies capitalizing on the high capacity of mammalian cells to produce insulinogenic components within a patient have gained increased attention because they promise effective drug production, delivery and dosage. For example, the regeneration of functional glucose-responsive insulin-secreting β-cells from stem cells (Pagliuca FW et al., Cell 159, 428-439 (2014)) represents a major breakthrough for treating T1D: transplantation of these *ex vivo* reprogrammed cells into T1D patients would directly restore their defective glucose-stimulated insulin expression. Approaches based on the delivery of glucose-responsive insulin expression elements into extrapancreatic mammalian cell types (Han J et al., WJG 18, 6420-6426 (2012)) can protect against fundamental diabetic vulnerabilities such as autoimmune (re)-targeting in T1D (Aguayo-Mazzucato C and Bonner-Weir S, Nat Rev Endocrinol 6, 139-148 (2010)) and metabolic stress-induced β-cell apoptosis in T2D (Marzban L et al., Diabetes 55, 2192-2201 (2006)). Recently, synthetic biology-inspired rational circuit design has led to the engineering of immunoprotective implants that enable trigger-inducible insulin- (Stanley S et al., Nat Med 21, 92-98 (2015)) or GLP1-expression (Ye H et al., Science 332, 1565-1568 (2011)) with traceless and non-invasive signals. However, neither of these approaches combines direct glucose sensing, endogenous real-time control of therapeutic dosage, and straightforward engineering of non-stem-cell human cells.

### SUMMARY OF THE INVENTION

The invention provides therapeutically applicable β-cell-mimetic cells and methods for producing such β-cell-mimetic cells. The β-cell-mimetic cells of the present invention comprise a carbohydrate-inducible transcriptional system that directly senses extracellular carbohydrate concentrations and is capable to coordinate the dose-dependent transcription of therapeutic proteins such as e.g. insulin and GLP-1. The system mimics core functions of pancreatic β-cells, which sense carbohydrates as glucose via a mechanism that combines glycolysis and stimulus-secretion coupling. Implanted β-cell-mimetic cells corrected insulin deficiency and self-sufficiently abolished persistent hyperglycaemia in T1D mice. Similarly, glucose-inducible GLP-1 transcription improved endogenous glucose-stimulated insulin release and glucose tolerance in T2D mice. The β-cell-mimetic cells of the present invention are useful for the treatment of metabolic diseases such as e.g. metabolic diseases selected from the group consisting of T1D, T2D, metabolic syndrome and cardiovascular disease.

Thus, in a first aspect, the invention relates to a recombinant cell comprising a nucleic acid construct comprising a promoter which is responsive to carbohydrate metabolism of said recombinant cell, wherein the promoter is operably linked to a gene coding for a therapeutic protein.

In a further aspect, the invention relates to an encapsulated cell comprising a recombinant cell.

In a further aspect, the invention relates to a recombinant cell or an encapsulated recombinant cell for use as a medicament.

In a further aspect, the invention relates to a recombinant cell or an encapsulated recombinant cell for use in a method for the prevention, delay of progression or treatment of a metabolic disease in a subject.

In a further aspect, the invention relates to a method of producing a recombinant cell expressing a therapeutic protein, said method comprising the steps of:
(a) obtaining a population of cells;
(b) transfecting said population of cells with a nucleic acid construct comprising a promoter which is responsive to a product of the carbohydrate metabolism of said cell, wherein the promoter is operably linked to a gene coding for a therapeutic protein;
(c) incubating the population of transfected cell in the presence of carbohydrates for a sufficient time to permit the transfected cells to express a therapeutic protein.

In a further aspect, the invention relates to a method to deliver a nucleic acid construct to a cell, wherein the nucleic acid construct comprises a promoter which is responsive to carbohydrate metabolism of said cell, wherein the promoter is operably linked to a gene coding for a therapeutic protein, comprising administering said nucleic acid construct to said cell, whereby said gene coding for a therapeutic protein is expressed in said cell in response to carbohydrate stimulation.

In a further aspect the present invention relates to a method for producing a therapeutic protein in vivo in a mammal, said method comprising:
(a) providing an in vitro population of recombinant cells into an implantable semi-permeable device;
(b) implanting the device with the cell population into a mammalian host; and
(c) maturing the cell population in said device in vivo such that at least some cells of the cell population are cells that produce a therapeutic protein in response to carbohydrate stimulation in vivo.

In a further aspect, the invention relates to an in vitro cell culture comprising the recombinant cell, wherein said recombinant cell is expressing a protein, preferably a therapeutic protein in the presence of carbohydrates.

### BRIEF DESCRIPTION OF THE DRAWINGS

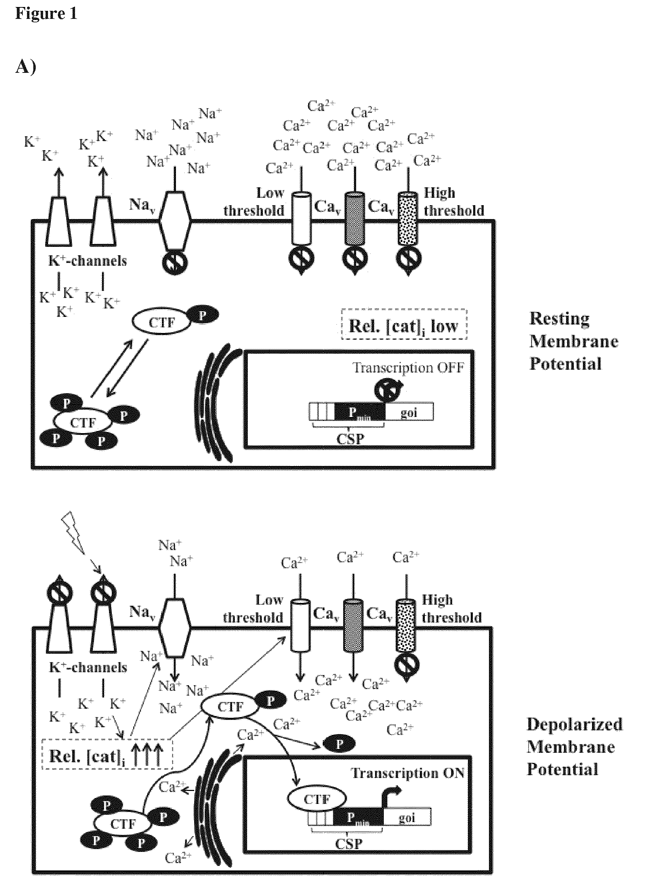

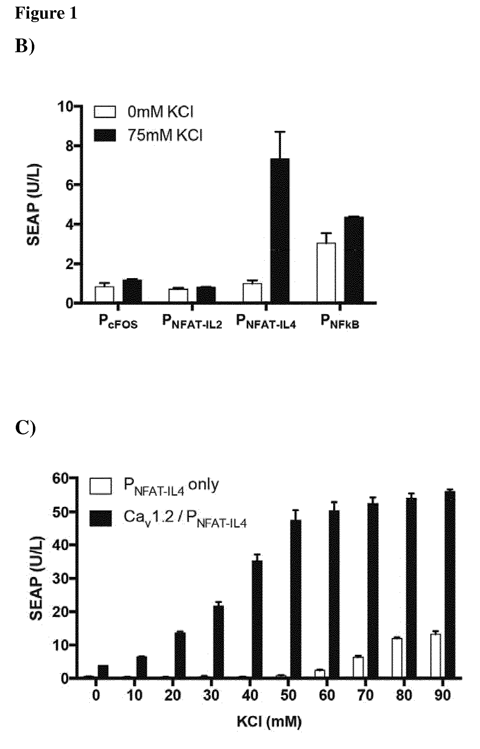

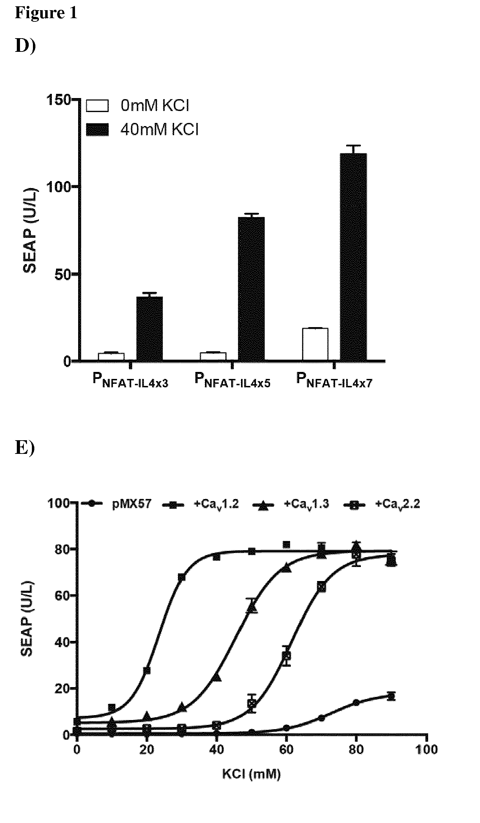

**Figure 1** shows engineering of an excitation-transcription coupling system in mammalian cells.
   **(A)** Scheme of a synthetic excitation-transcription coupling system. At resting membrane potentials, basic ion currents keep the relative concentration of intracellular cations (rel.[cat.]ᵢ) low and the plasma membrane hyperpolarized. Gene expression from calcium-specific promoters (CSPs) remains inactive. Stimuli that lead to a successive increase in intracellular cations either by blocking outward potassium channels (K⁺-channels) or by inducing the entry of cations result in membrane depolarization. Depending on the degree of depolarization and the subsequent instant membrane potential, different activation threshold-dependent voltage-gated sodium (Naᵥ) or calcium (Caᵥ) channels open and amplify the depolarization signal. Sustained increases of intracellular calcium levels activate various calcium-regulated transcription factors (CTFs) that translocate to the nucleus and initiate reporter gene transcription from synthetic cognate CSPs.
   **(B)** Calcium-specific promoters activated by chemically induced membrane depolarization. HEK-293 cells were transfected with pMX53 (P_{cFOS}-SEAP-pA), pHY30 (P_{NFAT-IL2}-SEAP-pA), pMX56 (P_{NFAT-IL4}-SEAP-pA) or pKR32 (P_{NFkB}-SEAP-pA) and grown in cell culture medium containing 0 or 75 mM potassium chloride (KCl). SEAP levels in the culture supernatants were profiled at 48 h after the addition of KCl. **(C)** Caᵥ1.2-amplified excitation-transcription coupling. HEK-293 cells were co-transfected with pMX56 (P_{NFAT-IL4}-SEAP-pA) and either Cav1.2 (pCaV1.2/pCavb3/pCaVα2δ1; 1:1:1, w/w) or pcDNA3.1(+), and the cells were grown for 48 h in cell culture medium containing different KCl concentrations before SEAP levels in the culture supernatants were profiled. **(D)** Optimization of the P_{NFAT-IL4}-promoter. HEK-293 cells were co-transfected with equal amounts of Caᵥ1.2 and SEAP expression vectors driven by promoter architectures containing three (pMX56, (NFAT_{IL4})₃-Pₘᵢₙ-SEAP-pA), five (pMX57, (NFAT_{IL4})₅-Pₘᵢₙ-SEAP-pA) or seven (pMX58, (NFAT_{IL4})₇-Pₘᵢₙ-SEAP-pA) mouse IL4-derived NFAT repeat (NFAT_{IL4}) sequences. Transfected cells were grown for 48 h in cell culture medium containing 0 or 40 mM KCl before SEAP levels in culture supernatants were profiled. **(E)** Activation threshold-dependent excitation-transcription coupling. HEK-293 cells were co-transfected with pMX57 and either pcDNA3.1(+), Caᵥ1.2 (pCaV1.2/pCavb3/pCaVα2δ1; 1:1:1, w/w), Caᵥ1.3 (pCaV1.3/pCavb3/pCaVα2δ1; 1:1:1, w/w) or Caᵥ2.2 (pCav2.2/pCavb3/pCaVα2δ1; 1:1:1, w/w), and the cells were grown for 48 h in cell culture medium containing different KCl concentrations before SEAP levels in the culture supernatants were profiled. All data presented are mean ± SD, n ≥ 5.

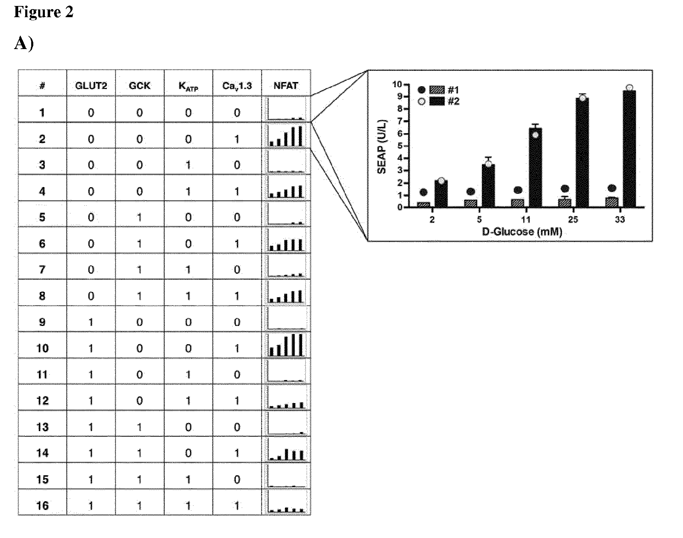

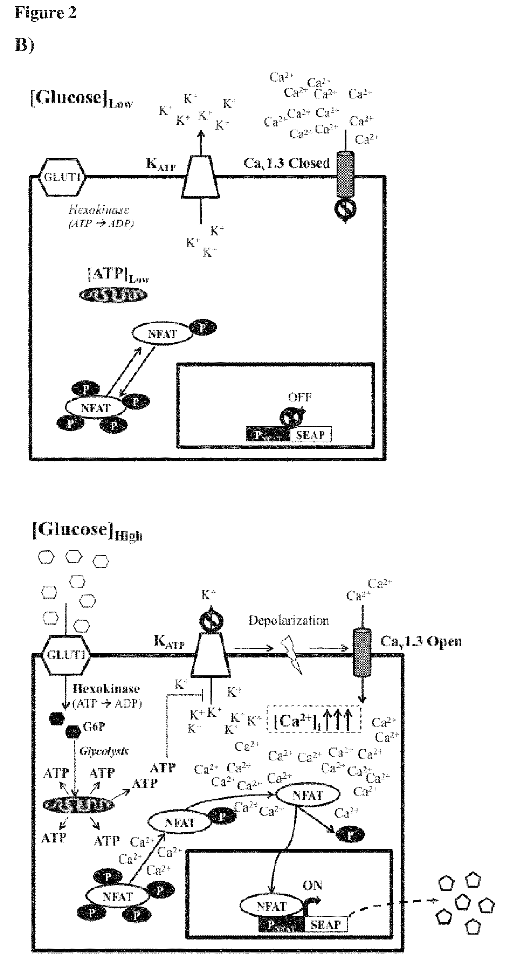

**Figure 2** shows glucose sensing in extrapancreatic human cells. **(A)** Contributory analysis of ectopically expressed glucose-sensing components in extrapancreatic human cells. HEK-293 cells were co-transfected with pMX57 and either (1) mammalian expression vectors for hGLUT2 (pcDNA3.2/v5-DEST hGlut2), GCK (pMX90), K_{ATP} (pCMV-hSUR1/pCMV6-hKir6.2; 1:1, w/w) and Caᵥ1.3 (pCaV1.3/pCavb3/pCaVα2δ1; 1:1:1, w/w) or (0): equal amounts of pcDNA3.1(+) (P_{hCMv}-MCS-pA). Twenty-four hours after transfection and cultivation in low glucose medium (2 mM), D-glucose was added to the indicated final concentrations. SEAP levels in the culture supernatants were scored at 48 h after the addition of D-glucose. Data presented are mean ± SD, n ≥ 5; Circles indicate simulation results. **(B)** Schematic representation of a hypothetical glucose-sensing mechanism in HEK-293 cells. Low levels of extracellular glucose are insufficient in inducing membrane depolarization to activate voltage-gated Caᵥ1.3 channels. By contrast, higher levels of extracellular glucose are taken up by mammalian cells to generate increased amounts of ATP. The subsequent closure of ATP-sensitive potassium channels (K_{ATP}) activates Caᵥ1.3, resulting in increased Ca²⁺ influx and the calcineurin-dependent activation of NFAT-regulated transcription units.

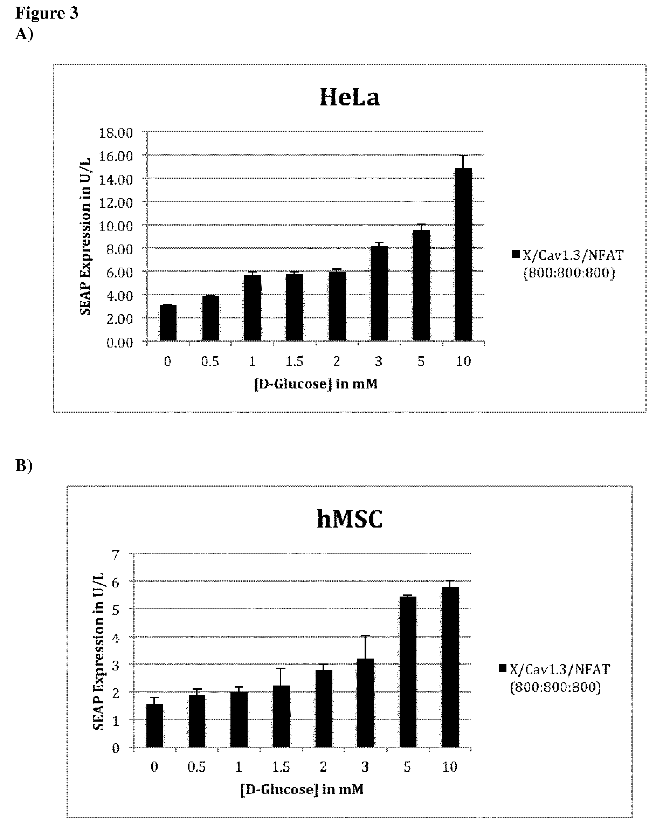

**Figure 3** shows Caᵥ1.3/P_{NFAT-IL4}-regulated SEAP expression in HeLa and human MSCs. (A) HeLa and (B) hMSCs were co-transfected with equal plasmid amounts of Caᵥ1.3 (pCaV1.3/pCavb3/pCaVα2δ1; 1:1:1, w/w), pMX57 and pcDNA3.1(+), and the cells were cultured in glucose-free medium for 12 h before different concentrations of D-glucose were added. Forty-eight hours after the addition of D-glucose, the SEAP levels in the culture supernatants were scored. Data presented are mean ± SD, n ≥ 3.

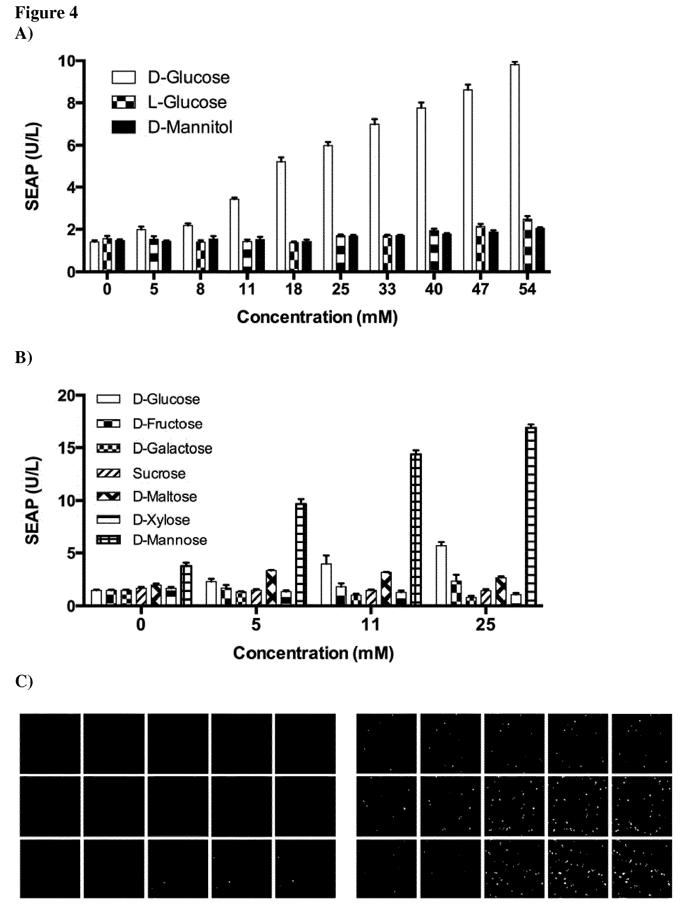

**Figure 4** shows characterization of Caᵥ1.3/P_{NFAT-IL4}-constituted excitation-transcription coupling systems. (A-B) Substrate specificity of Caᵥ1.3/pMX57-transgenic mammalian cells. HEK-293 cells were co-transfected with Caᵥ1.3 (pCaV1.3/pCavb3/pCaVα2δ1; 1:1:1, w/w) and pMX57, and the cells were cultured in glucose-free medium for 12 h before different **(A)** glucose isomers and osmotic controls or **(B)** nutritional sugar compounds were added. Forty-eight hours after the addition of control compounds, the SEAP levels in the culture supernatants were scored. Data presented are mean ± SD, n ≥ 3. (C) Right: Fluorescence micrographs profiling representative TurboGFP expression in HEK-293 cells co-transfected with Caᵥ1.3 and pFS119 ((NFAT_{IL4})₅-Pₘᵢₙ-TurboGFP:dest1-pA) and cultured in medium containing different concentrations of D-glucose (D-Glc) or potassium chloride (KCl). Left: Control cells transfected with pcDNA3.1(+) and pFS119.

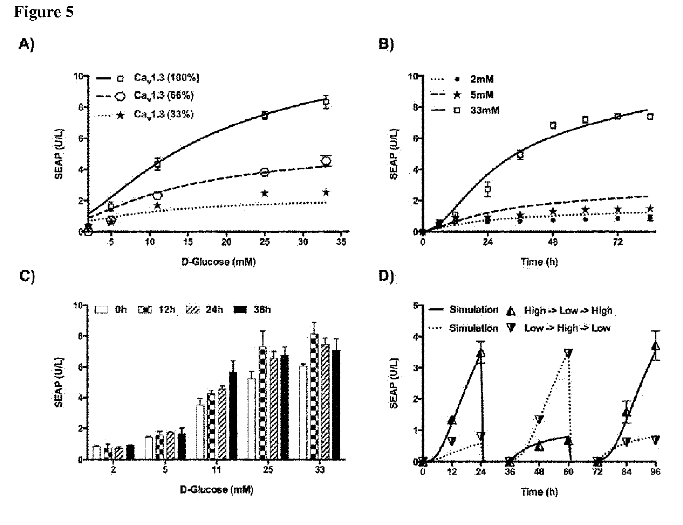

**Figure 5** shows characterization of the Caᵥ1.3-transgenic HEK-293_{NFAT-SEAP1} cell line. **(A)** Caᵥ1.3-dependent glucose sensing. Twenty-four hours after the transfection of HEK-29_{3NFAT}-_{SEAP1} cells with different amounts of Caᵥ1.3 expression vectors (pCaV1.3/pCavb3/pCaVα2δ1; 1:1:1, w/w) and cultivation in low-glucose medium (2 mM), D-glucose was added at the indicated final concentrations. The SEAP levels in the culture supernatants were scored at 48 h after the addition of D-glucose. Curves show corresponding simulations for data that was (100%) and was not (66% and 33%) used for calibrating the model. Data presented are mean ± SD, n ≥ 5. **(B)** SEAP expression kinetics. Twelve hours after cultivation in low-glucose medium (2 mM), Caᵥ1.3-transgenic HEK-293_{NFAT-SEAP1} cells were grown in cell culture medium containing different concentrations of D-glucose. The SEAP levels in the culture supernatants were profiled every 12 h. Solid and dashed curves show corresponding model-based simulations. Data presented are mean ± SD, n ≥ 5. (C) Time-delayed glucose responsiveness of the Caᵥ1.3/ HEK-293_{NFAT-SEAP1} system. Caᵥ1.3-transgenic Caᵥ1.3/HEK-293_{NFAT-SEAP1} cells were cultured in low-glucose medium (2 mM) for 0-36 h before D-glucose was added at the indicated final concentrations. Forty-eight hours after the addition of D-glucose, the SEAP levels in the culture supernatants were profiled. Data presented are mean ± SD, n ≥ 3. **(D)** Reversibility of the synthetic excitation-transcription coupling system. Caᵥ1.3-transgenic Caᵥ1.3/HEK-293_{NFAT-SEAP1} cells were cultured in high-D-glucose medium (40 mM) or low-D-glucose medium (5 mM) for 72 h while resetting the cell density to 0.75 x 10⁶ cells/mL and alternating D-glucose concentrations every 24 h followed by extensive washing over 12 h. The SEAP levels in the culture supernatants were profiled every 12 h within 24 h intervals of exposure to high/low glucose. Solid and dashed curves show corresponding model-based simulations. Data presented are mean ± SD, n ≥ 4.

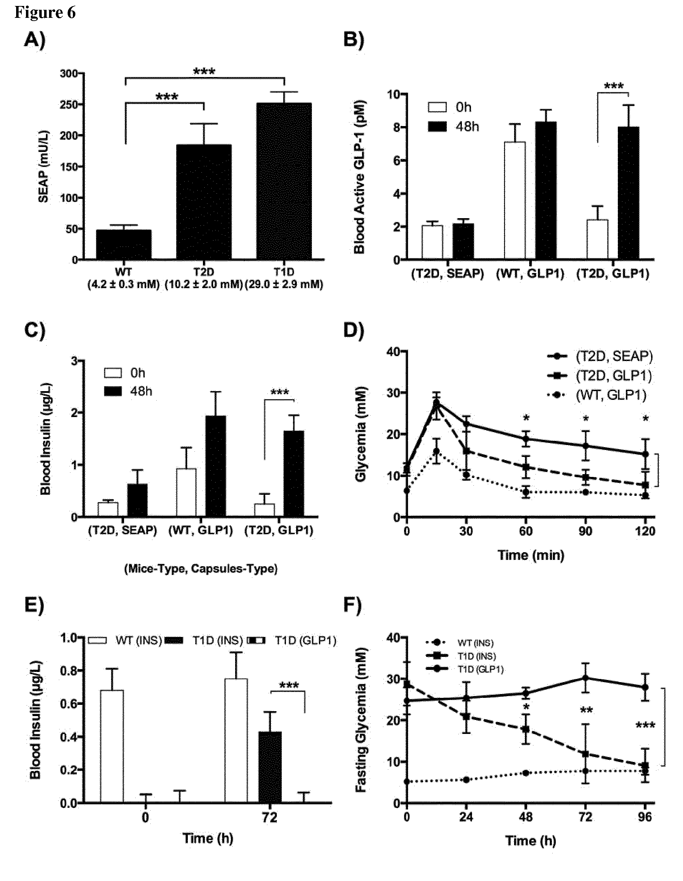

**Figure 6** shows Caᵥ1.3-dependent glucose sensing and antidiabetic potential in diabetic mice. **(A)** Dose-dependent glycaemia-induced SEAP expression in different diabetic mouse models. HEK-293_{NFAT-SEAP1} cells were transfected with Caᵥ1.3 and microencapsulated into alginate-poly-(L-lysine)-alginate beads. Capsules (1 x 10⁴; 500 cells/capsule) were implanted into mice suffering from different types of diabetes. The SEAP levels in the bloodstream of treated animals were quantified 48 h after implantation. **(B, C)** Self-sufficient GLP-1 expression in wild-type and type-2 diabetic mice. HEK-293 cells were co-transfected with Caᵥ1.3 and pMX115 ((NFAT_{IL4})₉-Pₘᵢₙ-shGLP1-pA), and the cells were then microencapsulated into alginate-poly-(L-lysine)-alginate beads (GLP-1 capsules). Control implants consisted of equally encapsulated Caᵥ1.3-transgenic HEK-293_{NFAT-SEAP1} cells (SEAP capsules). Capsules (1 x 10⁴; 500 cells/capsule) were implanted into wild-type (WT) or type-2 diabetic (T2D) mice. (B) GLP-1 and (C) insulin levels in the blood were profiled at 48 h after implantation. **(D)** Intraperitoneal glucose tolerance test (IPGTT) of wild-type and type-2 diabetic mice. Forty-eight hours after implantation and prior to serum collection, the same groups of mice as in (B and C) received an intraperitoneal injection of aqueous 2 g/kg D-glucose, and the glycaemic profile of each animal was tracked every 30 min. All data are shown as the mean ± SEM, and the analysis as performed with a two-tailed t-test (n = 8 mice). * P < 0.05, ** P < 0.01, *** P < 0.001 vs. control. **(E)** Self-sufficient insulin expression in wild-type and type-1 diabetic mice. HEK-293 cells were co-transfected with Caᵥ1.3 and pMX100 ((NFAT_{IL4})₉-Pₘᵢₙ-mINS-pA), and the cells were then microencapsulated into alginate-poly-(L-lysine)-alginate beads (INS capsules). Control implants consisted of equally encapsulated Caᵥ1.3/pMX115-transgenic HEK-293 cells (GLP-1 capsules). Capsules (1 x 10⁴; 500 cells/capsule) were implanted into wild-type (WT) or type-1 diabetic (T1D) mice. Serum insulin levels (detection limit: 0.2 µg/L) were profiled at 72 h after capsule implantation and 4 h after food intake. **(F)** Self-sufficient glycaemic control of Caᵥ1.3/pMX100-transgenic implants. The fasting glycaemia of the same groups of mice as in (E) was tracked for 96 h after capsule implantation. All data in (E-F) are shown as the mean ± SEM, and the statistical analysis was performed with a two-tailed t-test (n = 8 mice). * P < 0.05, ** P < 0.01, *** P < 0.001 vs. control.

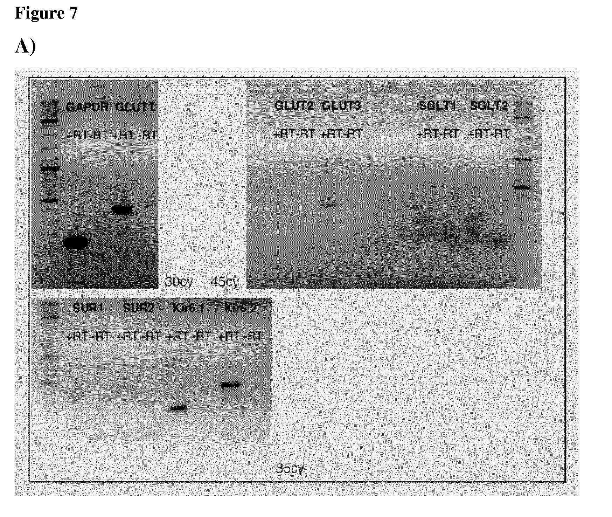

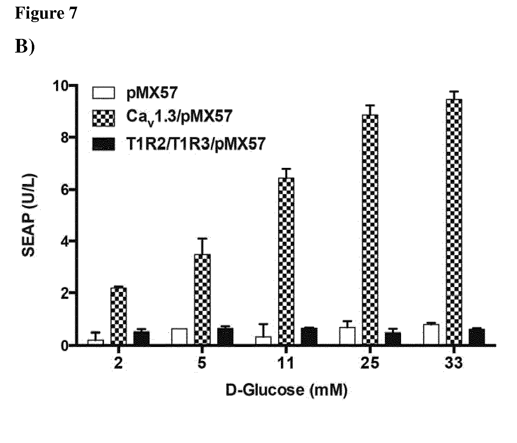

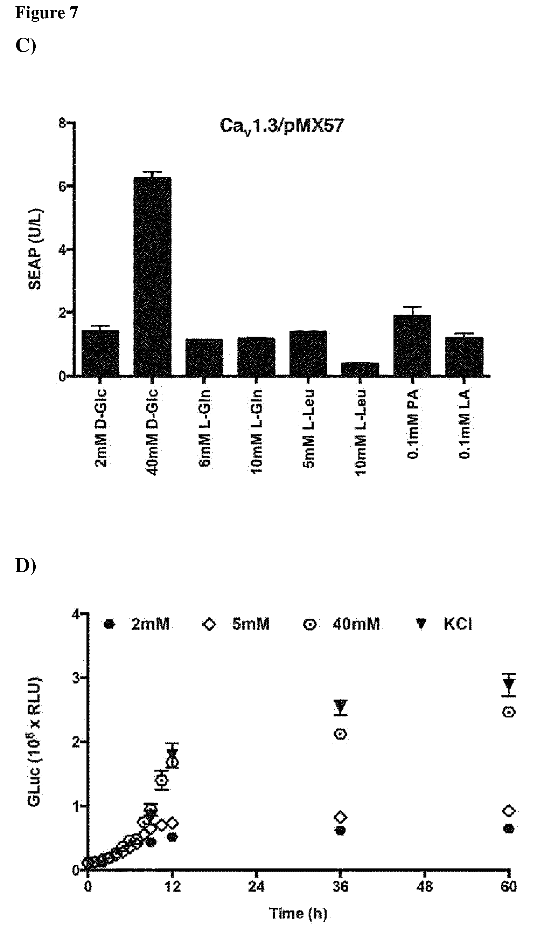

**Figure 7** shows glucose-sensor control experiments. (A) Semi-quantitative RT-PCR-based transcriptional profiling of endogenously expressed glucose transporters and K_{ATP}-subunits in HEK-293. cDNA of untreated HEK-293 cells was generated by RNA isolation and reverse transcription with MultiScribe Reverse Transcriptase (RT). Amplicons of target components were generated by subsequent PCR-reactions of different cycles with specific primers shown in Table 2. (B) Comparison of different glucose sensors in mammalian cells. HEK-293 cells were co-transfected with 1000ng pMX57 and 1000ng of either pcDNA3.1(+), Caᵥ1.3 (pCaV1.3 / pCaVb3 / pCaVα2δ1; 1:1:1, w/w) or the full sweet taste receptor componentry (pT1R2 / pT1R3 / pGNAT3; 1:1:1, w/w). 24h after transfection and cultivation in low glucose medium (2mM), D-glucose was added to final concentrations as indicated on the x-axis. SEAP levels in the culture supernatants were scored at 48h after addition of D-glucose. Data presented are mean ± SD, n ≥ 5. (C) Substrate specificity of Caᵥ1.3/pMX57-transgenic mammalian cells. HEK-293 cells were co-transfected with Caᵥ1.3 (pCaV1.3 / pCaVb3 / pCaVα2δ1; 333ng each) and pMX57 (1000ng) and cultivated in glucose-free medium for 12h before different concentrations of D-glucose (D-Glc), glutamine (L-Gln), leucine (L-Leu), palmitic acid (PA) or linoleic acid (LA) were added. 48h after addition of control compounds, SEAP levels in the culture supernatants were scored. Data presented are mean ± SD, n ≥ 3. (D) GLuc expression kinetics. HEK-293 cells were co-transfected with Caᵥ1.3 (pCaV1.3 / pCaVb3 / pCaVα2δ1; 333ng each) and pWH29 ((NFAT_{IL4})₅-Pₘᵢₙ-GLuc-pA; 1000ng) and cultivated in low-glucose medium (2mM) for 12h before different concentrations of D-Glucose (5 or 40mM) or KCl (40mM) were added. GLuc levels in the culture supernatants were profiled at different time points after the addition of inducer compounds as indicated on the x-axis. All data presented are mean ± SD, n ≥ 3.

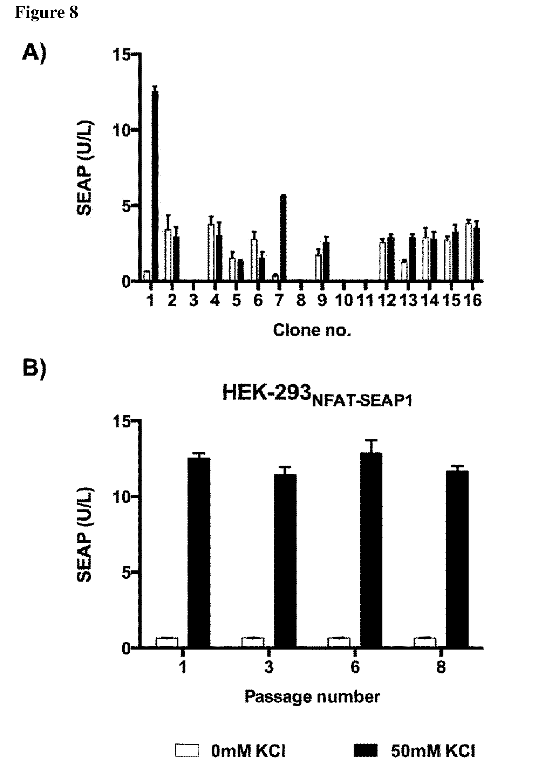

**Figure 8** shows design and construction of the stable HEK-_{293NFAT-SEAP1} cell line. (A) Depolarization-stimulated SEAP expression of different P_{NFAT-IL4}-transgenic cell clones (HEK-293_{NFAT}-_{SEAP1}). HEK-293 cells were stably transfected with pMX57 ((NFAT_{IL4})₅-Pₘᵢₙ-SEAP-pA) and 16 randomly selected cell clones were profiled for their depolarization-stimulated SEAP regulation performance by cultivating them for 48h in the presence (50mM) or absence (0mM) of potassium chloride (KCl). (B) Stable depolarization-stimulated SEAP expression of the HEK-293_{NFAT-SEAP1} cell line. 5x 10⁴ HEK-293_{NFAT-SEAP1} cells from different generations were cultivated for 48h in the presence (50mM) or absence (0mM) of potassium chloride (KCl) before SEAP levels in the culture supernatants were scored. All data presented are mean ± SD, n ≥ 3.

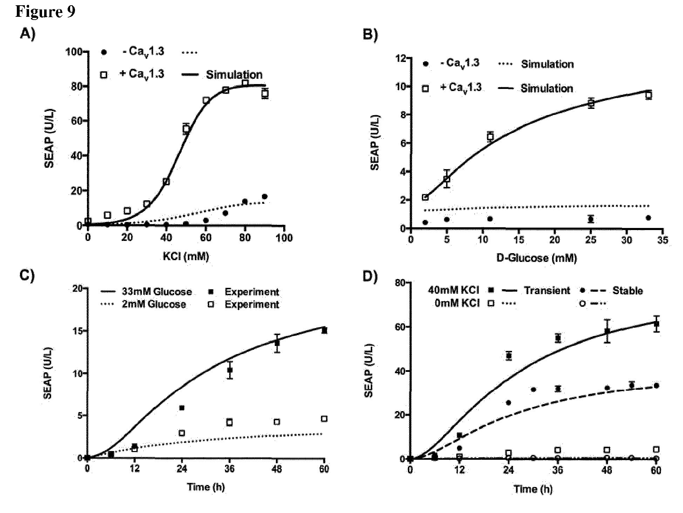

**Figure 9** shows experimental data (symbols) and simulation results (lines) for D-glucose- and KCl-stimulated SEAP expression in vitro. (A) Cav1.3-dependent excitation-transcription coupling. HEK-293 cells were co-transfected with pMX57 and either pcDNA3.1(+) (-Caᵥ1.3) or Caᵥ1.3 (+Caᵥ1.3), and the cells were grown for 48 h in cell culture medium containing different KCl concentrations before SEAP levels in the culture supernatants were profiled (see also Fig. 1E). All data presented are mean ± SD, n ≥ 5. (B) D-glucose activated P_{NFAT3}-activation. HEK-293 cells were co-transfected with Caᵥ1.3 and pMX57, and the cells were cultured in low-glucose medium (2 mM) for 12 h before D-glucose was added to the indicated final concentrations. Forty-eight hours after the addition of control compounds, the SEAP levels in the culture supernatants were scored. Data presented are mean ± SD, n ≥ 3. (C, D) SEAP expression kinetics. Twelve hours after transfection of 9 x 10⁵ HEK-293 cells with pMX57 (P_{NFAT3}-SEAP-pA) and Caᵥ1.3 (pCaV1.3/pCaVb3/pCaVα2δ1; 1:1:1, w/w), culture supernatants were exchanged by fresh medium containing different D-glucose- (C) and KCl- (D) concentrations. SEAP levels in the culture supernatants were profiled every 12 h. Circles represent equally treated Caᵥ1.3-transgenic HEK-_{293NFAT-SEAP1} cells. Data presented are mean ± SD, n ≥ 3.

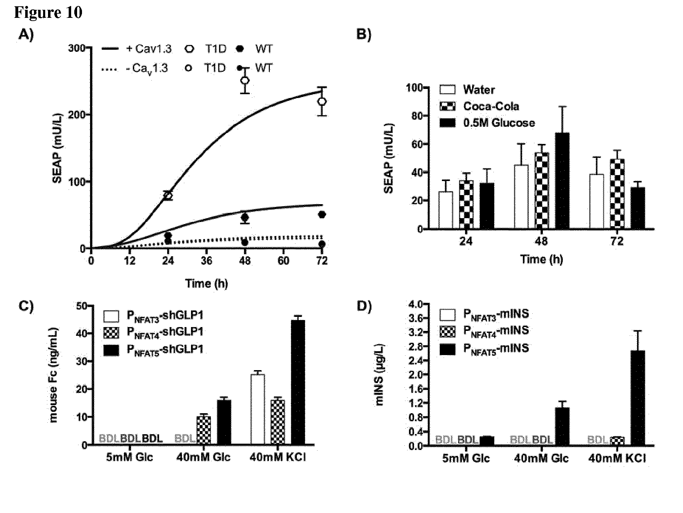

**Figure 10** shows glucose-sensor in vivo control experiments. (A) Caᵥ1.3-dependent SEAP-expression kinetics in vivo. Wild-type (WT, black) or type 1 diabetic (T1D, white) mice were implanted with 1 x 10⁴ microencapsules (500 cells /capsule) containing HEK-293_{NFAT-SEAP1} cells transfected with either pcDNA3.1 (+) (-Caᵥ1.3) or Caᵥ1.3 (+Caᵥ1.3), and SEAP levels in the bloodstream were quantified every 24h (opaque or hollow circles). Solid or dashed curves show corresponding model-based simulations. The data are shown as the mean ± SD, n=8 mice. (B) Diet-induced glucose sensing in vivo. Wild-type mice were implanted with 1 x 10⁴ microcapsules (500 x Caᵥ1.3- transgenic HEK-293_{NFAT-SEAP1} cells/capsule) and received 4x daily oral administrations of 200µl water, Coca-Cola^{®} or aqueous D-glucose (0.5M). SEAP levels in the bloodstream were quantified every 24h after capsules implantation. The data are shown as the mean ≥ SD, n=8 mice. (C, D) Optimization of the P_{NFAT}-IL4-promoter for glucose- and depolarization-stimulated (C) shGLP1- and (D) mINS-expression. (C) HEK-293 cells were co-transfected with 1000ng Caᵥ1.3 and 1000ng of pMX61 ((NFAT_{IL4})₅-Pₘᵢₙ-shGLP1-pA), pMX117 ((NFAT_{IL4})₇-Pₘᵢₙ-shGLP1-pA) or pMX115 ((NFAT_{IL4})₉-Pₘᵢₙ-shGLP1-pA) and cultivated in low-glucose medium (2mM) for 12h before different concentrations of D-glucose (Glc) or potassium chloride (KCl) were added. 48h after addition of control compounds, murine IgG levels in the culture supernatants were quantified (BDL: below detection limit of 9.375ng/mL). (D) HEK-293 cells were co-transfected with 1000ng Caᵥ1.3 and 1000ng of pMX68 ((NFAT_{IL4})₅-Pₘᵢₙ-mINS-pA), pMX99 ((NFAT_{IL4})₇-Pₘᵢₙ-mINS-pA) or pMX100 ((NFAT_{IL4})₉-Pₘᵢₙ-mINSpA) and cultivated in low-glucose medium (2mM) for 12h before different concentrations of D-glucose (Glc) or potassium chloride (KCl) were added. 48h after addition of control compounds, murine insulin levels in the culture supernatants were quantified (BDL: below detection limit of 0.2µg/L). All data presented are mean ± SD, n ≥ 3.

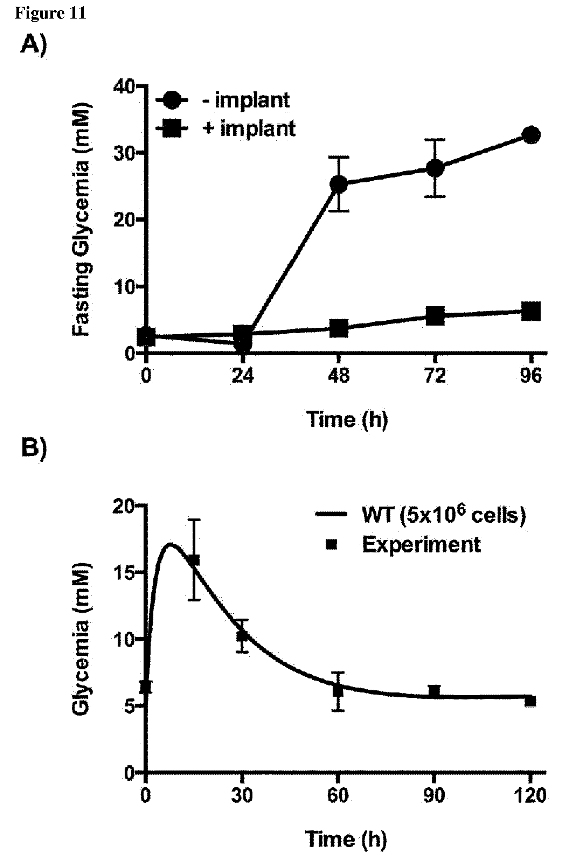

**Figure 11** shows (A) Glycaemic control in healthy and T1D mice. (Circles) Fasting CD-1 Swiss albino mice (2x 18h/day) were injected with a single dose of freshly diluted alloxan monohydrate (ALX; 200 mg/kg in 300µL phosphate buffered saline) and fasting glycaemia was measured every 24h after ALX injection. (Squares) Equally treated CD-1 Swiss albino mice harboring implants containing 5 x 10⁶ microencapsulated Cav1.3/pMX100-transgenic HEK-293 cells. Fasting glycaemia data are shown as the mean ± SD, n = 6 mice. (B) Model simulations for glucose tolerance in healthy WT-mice. Forty-eight hours after implantation of 5 x 10⁶ Caᵥ1.3 transgenic HEK-293_{NFAT-SEAP1} cells, CD-1 Swiss albino mice received an intraperitoneal injection of aqueous 2 g/kg D-glucose, and the glycaemic profile of each animal was tracked every 30 min. The curve shows a corresponding model-based simulation. All data shown as the mean ± SD, n = 8 mice.

### DETAILED DESCRIPTION

So that the invention may be more readily understood, certain terms are first defined. Unless otherwise defined within the specification, all technical and scientific terms used herein have their art-recognized meaning. Although similar or equivalent methods and materials to those described herein can be used in the practice or testing of the invention, suitable methods and materials are described below. All publications, patent applications, patents, and other references mentioned herein are incorporated by reference in their entirety. In case of conflict, the present specification, including definitions, will prevail. The materials, methods, and examples are illustrative only and not intended to be limiting. The terms "comprising", "having", and "including" are to be construed as open-ended terms (i.e., meaning "including, but not limited to,") unless otherwise noted.

As used herein, the term "beta-cell" or "β-cell" refers to a cell type found in the pancreas, in particular in the mammalian, more particular in the human pancreatic islets. Beta cells are the primary producers of insulin.

As used herein, the term "recombinant cell" refers to cells, preferably mammalian cells , more preferably human cells, which have been artificially manipulated to express genes which are introduced to the mammalian cells by e.g. transfection or transformation using nucleic acid constructs, such as e.g. expression vectors in which those genes are incorporated.

As used herein, the term "nucleic acid construct" refers to a nucleic acid, preferably to a recombinant nucleic acid construct, i.e. a genetically engineered nucleic acid construct which includes the nucleic acid of a gene and at least one promoter for directing transcription of the nucleic acid in a host cell. Nucleic acid constructs of the present invention are preferably suitable for mammalian cell expression. The nucleic acid construct (also referred to herein as an "expression vector") may include additional sequences that render the construct e.g. the vector, suitable for replication and integration in eukaryotes (e.g. shuttle vectors). In addition, a typical nucleic acid construct such as e.g. a cloning vector may also contain transcription and translation initiation sequences, transcription and translation terminators, and a polyadenylation signal.

As used herein, the term "promoter" refers to a regulatory DNA sequence generally located upstream of a gene that mediates the initiation of transcription by directing RNA polymerase to bind to DNA and initiating RNA synthesis. The term "Pₘᵢₙ" as used herein refers to a minimal promoter, preferably to the promoter as shown in SEQ ID NO: 33. A minimal promoter usually does not contain an enhancer i.e. do not comprise enhancer elements and is not a constitutive promoter. Preferably a minimal promoter shows no or only minimal transcriptional activity in the absence of transcription factors.

As used herein, the term "enhancer" as used herein refers to a nucleotide sequence that acts to potentiate the transcription of genes independent of the identity of the gene, the position of the sequence in relation to the gene, or the orientation of the sequence.

As used herein, the terms "functionally linked" and "operably linked" are used interchangeably and refer to a functional relationship between two or more DNA segments, in particular gene sequences to be expressed and those sequences controlling their expression. For example, a promoter and/or enhancer sequence, including any combination of cis-acting transcriptional control elements is operably linked to a coding sequence if it stimulates or modulates the transcription of the coding sequence in an appropriate host cell or other expression system.

Promoter regulatory sequences that are operably linked to the transcribed gene sequence are physically contiguous to the transcribed sequence.

As used herein, the term "promoter which is responsive to carbohydrate metabolism of a cell" refers to a promoter which is activated or repressed for transcription in response to the presence or absence of a product of the carbohydrate metabolism of a cell. A product of the carbohydrate metabolism of a cell can be a metabolic product of the cellular pathway of carbohydrate transformation or carbohydrate degradation e.g a product of glycolysis such as intracellular ATP production or can be a cellular response to the metabolism of a carbohydrate in the cell e.g. production of intracellular ATP lead to the closure of K_{ATP} channels which causes membrane depolarization as cellular response.

As used herein, the term "carbohydrate metabolism of a cell" refers to the various biochemical processes responsible for the formation, breakdown and interconversion of carbohydrates in living organisms. Oligosaccharides and/or polysaccharides are typically cleaved into smaller monosaccharides by enzymes called glycoside hydrolases. The monosaccharide units then enter the cellular pathway of carbohydrate transformation or carbohydrate degradation, i.e. the cellular pathway of glucose transformation or degradation such as glycolysis.

As used herein, the term "cellular component for sensing extracellular carbohydrates" refers to a cellular component such as e.g. a transporter of carbohydrates like e.g. a transporter of glucose or a glucose linked transporter, a receptor involved in the regulation of carbohydrate homeostasis e.g. glucose homeostasis, or a membrane protein like potassium channels, calcium channels or sodium channels which is capable of sensing extracellular carbohydrates such as glucose by activating a gene, preferably a gene coding for a therapeutic protein whose expression level correlates with the extracellular carbohydrate levels. Usually the cellular component for sensing extracellular carbohydrates of the recombinant cell of the present invention is capable of sensing extracellular carbohydrates such as glucose by activating a gene, preferably a gene coding for a therapeutic protein whose expression level correlates with the extracellular carbohydrate levels via a promoter which is responsive to carbohydrate metabolism of said recombinant cell, wherein the promoter is operably linked to a gene coding for the therapeutic protein. Preferably the cellular component for sensing extracellular carbohydrates is of mammalian, more preferably human origin.

As used herein, the term "carbohydrate" or "saccharide" are interchangeably and equivalently used within this context refer to a biological molecule consisting of carbon (C), hydrogen (H) and oxygen (O) atoms, usually with a hydrogen-oxygen atom ratio of 2:1 (as in water); in other words, with the empirical formula Cₘ(H₂O)ₙ (where m could be different from n). Carbohydrates include monosaccharides, disaccharides, oligosaccharides, and polysaccharides, preferably monosaccharides and disaccharides, more preferably monosaccharides, most preferably monosaccharides selected from the group consisting of glucose, galactose, fructose and xylose and epimeric forms thereof like mannose, in particular glucose and mannose. The term "glucose" in its broadest sense relates to glucose and its epimeric forms like mannose. Preferably the term "glucose" relates to glucose (D-Glucose, (2R,3S,4R,5R)-2,3,4,5,6-Pentahydroxyhexanal).

As used herein, the term "membrane protein" refers to a protein molecule that is attached to or associated with the membrane of a cell or organelle. The membrane protein is preferably a membrane protein of a human cell or organelle.

As used herein, the term "fragment of a membrane protein" refers to a region of a membrane protein that is shorter in length as compared with the full-length membrane protein. It is however, a requirement of the present invention that any fragment of a membrane protein used as part retain the activity of the full-length membrane protein.

As used herein, the term "subunit of a membrane protein" or "monomer of a membrane protein" are interchangeably and equivalently used within this context and refers to a separate polypeptide chain that makes a membrane protein which is made up of two or more polypeptide chains joined together. In a membrane protein molecule composed of more than one subunit, each subunit can form a stable folded structure by itself. The amino acid sequences of subunits of a protein can be identical, similar, or completely different.

As used herein, the term "physiological effect of membrane depolarization" refers to a physiological effect due to depolarization of a cell's membrane such as e.g. the variation in intra-cellular cation, e.g. intra-cellular calcium, intra-cellular sodium or intra-cellular potassium concentration, in particular intra-cellular calcium concentration within a cell which has been caused by physiological activities, in particular by the carbohydrate metabolism of said cell.

As used herein, the term "calcium-responsive promoter" refers to a promoter which is activated or repressed for transcription in response to the presence or absence of calcium in the cell.

As used herein, the term "expression system" refers to a set of transgenic genetic elements within a cell as well as proteins encoded by such genetic elements.

The term "G-protein coupled receptors" as used herein refers to a family of transmembrane receptors, that sense molecules outside the cell and activate responses inside the cell by coupling with specific intracellular signaling pathways via G proteins. Preferred G-protein coupled receptors are GPR 1, TAS1R2, TAS1R3, GLP1R or anyone of their orthologues. Most preferred G-protein coupled receptors are GPR1 (GenBank: CAA98593.1), TAS1R2 (UniProtKB/Swiss-Prot: Q8TE23.2), TAS1R3 (UniProtKB/Swiss-Prot: Q7RTX0.2) and GLP1R (UniProtKB/Swiss-Prot: P43220.2).

The term "SLC2A family glucose transporters" (also known as GLUT) as used herein refers to a family of transmembrane proteins that catalyze the entry of carbohydrates into mammalian cells. Preferred SLC2A family glucose transporters are GLUT1 (SLC2A1), GLUT2 (SLC2A2), GLUT3 (SLC2A3), GLUT4 (SLC2A4), GLUT5 (SLC2A5), GLUT6 (SLC2A6), GLUT7 (SLC2A7), GLUT8 (SLC2A8), GLUT9 (SLC2A9), GLUT10 (SLC2A10), GLUT11 (SLC2A11), GLUT12 (SLC2A12) and GLUT13 (SLC2A13) or anyone of their orthologues. Most preferred SLC2A family glucose transporters are human GLUT1, human GLUT2 and human GLUT3.

The term "SLC5A family sodium-glucose linked transporters" (also known as SGLT) as used herein refers to a family of transmembrane proteins that mediate sodium-dependent co-transport of carbohydrates across the plasma membrane of mammalian cells. Preferred SLC5A family sodium-glucose linked transporters are SGLT1 (SLCSA1), SGLT2 (SLC5A2) and SGLT3 (SLC5A3) or anyone of their orthologues. Most preferred SLC5A family sodium-glucose linked transporters are human SGLT1 and human SGLT3.

The term "potassium channels" as used herein refers to a family of pore-forming transmembrane proteins that facilitate the transport of potassium ions across the cell plasma membrane. Preferred potassium channels are ATP-sensitive potassium channels (K_{ATP}), calcium-activated potassium channels (BK_{Ca}), inward rectifier potassium channels (Kir) and voltage-dependent potassium channels (Kᵥ). Most preferred potassium channels are human ATP-sensitive potassium channels (K_{ATP}), human calcium-activated potassium channels (BK_{Ca}), human inward rectifier potassium channels (Kir) and human voltage-dependent potassium channels (Kᵥ).

The term "calcium channels" as used herein refers to a family of pore-forming transmembrane proteins that facilitate the transport of calcium ions across the cell plasma membrane. Preferred calcium channels are voltage-gated calcium channels (VGCC), N-methyl-D-aspartate type of Glutamate (NMDA) receptors, Ca²⁺ release-activated Ca²⁺ current (CRAC) channels and transient receptor potential channels (TRPCs). Most preferred calcium channels are human voltage-gated calcium channels (VGCC), human N-methyl-D-aspartate type of Glutamate (NMDA) receptors, human Ca²⁺ release-activated Ca²⁺ current (CRAC) channels and human transient receptor potential channels (TRPCs).

The term "sodium channels" as used herein refers to a family of pore-forming transmembrane proteins that facilitate the transport of sodium ions across the cell plasma membrane. Preferred sodium channels are voltage gated-sodium channels, most preferably human voltage gated-sodium channels

As used herein, the term "voltage-gated calcium channel" (VDCC) refers to a group of voltage-gated ion channels, preferably human voltage-gated calcium channels, found in the membrane of excitable cells whose permeability to the calcium ion Ca²⁺ correlates with the membrane potential. Voltage-dependent calcium channels are formed as a complex of several different subunits. Subunits known are the pore-forming Caᵥα1, the intracellular Caᵥβ, the transmembrane Caᵥγ, and a disulfide-linked dimer Caᵥα2δ .The α1 subunit is the primary subunit necessary for channel functioning in the VDCC, and consists of the characteristic four homologous I-IV domains containing six transmembrane α-helices each forms the ion conducting pore while the associated subunits have several functions including modulation of gating. Voltage-gated calcium channels are functionalized by their α1 subunit, which sets the activation threshold of the entire channel. Non-limiting examples of α1 subunits are Cav1.1, Caᵥ1.2, Caᵥ1.3, Caᵥ1.4, Caᵥ2.1, Caᵥ2.2, Caᵥ2.3, Caᵥ3.1, Caᵥ3.2 and Caᵥ3.3.

As used herein, the term "therapeutic protein" refers to a protein which is therapeutically applicable i.e. a therapeutic protein is any protein or polypeptide that can be expressed to provide a therapeutic effect, in particular a protein that can be expressed to provide a therapeutic effect with respect to metabolic diseases.

As used herein, the term "orthologues " with respect to a protein e.g. a receptor or channel refers to one of two or more homologous gene sequences found in different species.

As used herein, the term "insulin" refers to the protein hormone produced by beta cells in the pancreas which decreases blood glucose concentrations and is therefore involved in the regulation of blood sugar levels. One international unit of insulin (1 IU) is defined as the "biological equivalent" of 34.7 µg pure crystalline insulin, which corresponds to the amount required to reduce the concentration of blood glucose in a fasting rabbit to 45 mg/dl (2.5 mmol/L). Insulin is produced as a proinsulin precursor consisting of the B and A chains of insulin linked together via a connecting C-peptide. Insulin itself is comprised of only the B and A chains. Human insulin is encoded by the INS gene corresponding to GenBank Accession No: NM-000207.2. The term "insulin" or "insulin molecule" is a generic term that designates the 51 amino acid heterodimer comprising the A-chain peptide and the B-chain peptide, wherein the cysteine residues a positions 6 and 11 of the A chain are linked in a disulfide bond, the cysteine residues at position 7 of the A chain and position 7 of the B chain are linked in a disulfide bond, and the cysteine residues at position 20 of the A chain and 19 of the B chain are linked in a disulfide bond. The term "insulin" means the active principle of the pancreas that affects the metabolism of carbohydrates in the animal body and which is of value in the treatment of diabetes mellitus. The term includes synthetic and biotechnologically derived products that are the same as, or similar to, naturally occurring insulins in structure, use, and intended effect and are of value in the treatment of diabetes mellitus.

The term "insulin analogue" as used herein includes any heterodimer analogue or single-chain analogue that comprises one or more modification(s) of the native A-chain peptide and/or B-chain peptide. Modifications include but are not limited to substituting an amino acid for the native amino acid at a position selected from A4, A5, A8, A9, A10, A12, A13, A14, A15, A16, A17, A18, A19, A21, B1, B2, B3, B4, B5, B9, B10, B13, B14, B15, B16, B17, B18, B20, B21, B22, B23, B26, B27, B28, B29, and B30; deleting any or all of positions B 1-4 and B26-30; or conjugating directly or by a polymeric or non-polymeric linker one or more acyl, polyethylglycine (PEG), or saccharide moiety (moieties); or any combination thereof. Examples of insulin analogues include but are not limited to the heterodimer and single-chain analogues disclosed in published international application WO20100080606, WO2009099763, and WO2010080609, the disclosures of which are incorporated herein by reference. Examples of single-chain insulin analogues also include but are not limited to those disclosed in published International Applications WO9634882, WO95516708, WO2005054291, WO2006097521, WO2007104734, WO2007104736, WO2007104737, WO2007104738, WO2007096332, WO2009132129; U.S. Pat. Nos. 5,304,473 and 6,630,348; and Kristensen et al., Biochem. J. 305: 981-986 (1995), the disclosures of which are each incorporated herein by reference. The term "insulin analogues" further includes single-chain and heterodimer polypeptide molecules that have little or no detectable activity at the insulin receptor but which have been modified to include one or more amino acid modifications or substitutions to have an activity at the insulin receptor that has at least 1%, 10%, 50%, 75%, or 90% of the activity at the insulin receptor as compared to native insulin and which further includes at least one N-linked glycosylation site. In particular aspects, the insulin analogue is a partial agonist that has from 2× to 100× less activity at the insulin receptor as does native insulin. In other aspects, the insulin analogue has enhanced activity at the insulin receptor, for example, the IGF<B16B17>derivative peptides disclosed in published international application WO2010080607 (which is incorporated herein by reference). These insulin analogues, which have reduced activity at the insulin growth hormone receptor and enhanced activity at the insulin receptor, include both heterodimers and single-chain analogues.

As used herein, the term "autologous" or "endogenous" refers to any material that is present in a cell or an organism which is native to said recombinant cell or organism.

The term "stem cell" as used herein refers to undifferentiated biological cells that can differentiate into specialized cells and which is capable of proliferation to produce more stem cells.

As used herein, the term "somatic cell" refers to any cell forming the body of an organism, as opposed to germline cells or undifferentiated stem cells.

The terms "individual," "subject" or "patient" are used herein interchangeably. In certain embodiments, the subject is a mammal. Mammals include, but are not limited to primates (including human and non-human primates). In a preferred embodiment, the subject is a human.

The term "about" as used herein refers to +/- 10% of a given measurement.

In a first aspect, the present invention provides a recombinant cell comprising a nucleic acid construct comprising a promoter which is responsive to carbohydrate metabolism of said cell, wherein the promoter is operably linked to a gene coding for a therapeutic protein. Preferably, the promoter which is responsive to carbohydrate metabolism is responsive to glucose metabolism.

In one embodiment the promoter which is responsive to carbohydrate metabolism is responsive to a physiological effect of membrane depolarization caused by the carbohydrate metabolism of said recombinant cell. Preferably the physiological effect of membrane depolarization caused by the carbohydrates metabolism of the cell is extracellular calcium influx. Thus in a preferred embodiment the promoter which is responsive to carbohydrate metabolism is responsive to a physiological effect of membrane depolarization caused by the carbohydrate metabolism of said recombinant cell, wherein the physiological effect of membrane depolarization caused by the carbohydrates metabolism of said cell is extracellular calcium influx. Thus in a further preferred embodiment the promoter which is responsive to carbohydrate metabolism is responsive to extracellular calcium influx.

In one embodiment the promoter which is responsive to carbohydrate metabolism is a calcium-responsive promoter, preferably a calcium-responsive promoter comprising nucleic acid sequences bound by transcription factors of the NFAT family, the NFkB family, the AP-1 family, and/or the CREB family and/or cFOS, more preferably a calcium-responsive promoter comprising nucleic acid sequences bound by transcription factors of the NFAT family.

The NFAT family is a family of transcription factors shown to be important in immune response. One or more members of the NFAT family is expressed in most cells of the immune system. NFAT is also involved in the development of cardiac, skeletal muscle, and nervous systems. The NFAT family comprises the NFAT1, NFAT2, NFAT3, NFAT4, and NFAT5 proteins that specifically bind their cognate promoters. Preferred promoters that contain NFAT-binding sites are synthetic or natural promoters containing one or multiple 5'-GGAAA-3' consensus sites, more preferred are mammalian cytokine promoters e.g. mammalian cytokine promoters selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPARγ promoter. Most preferred is the murine interleukin (IL)-4 promoter. Thus in one embodiment the promoter which is responsive to carbohydrate metabolism of the recombinant cell is a synthetic or natural promoter that contains NFAT-binding sites containing one or multiple 5'-GGAAA-3' consensus sites, more preferably a mammalian cytokine promoter e.g. a mammalian cytokine promoter selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPARγ promoter and most preferrably the murine interleukin (IL)-4 promoter. The murine interleukin (IL)-4 promoter is described e.g. in Rooney JW et al., EMBO J 13, 625-633 (1994).

The NFkB family is as family of protein complexes that act as transcription factors controlling the transcription of DNA, cytokine production and cell survival. The NFkB family comprises NF-κB1, NF-κB2, RelA, RelB and c-Rel. Preferred members of the NFkB family are NF-κB1 and NF-κB2, more preferably human NF-κB1 and NF-κB2.

The AP-1 family is as family of transcription factors that regulates gene expression in response to a variety of stimuli, including cytokines, growth factors, stress and infections. AP-1 is a heterodimer composed of proteins belonging to the c-Fos, c-Jun, ATF and JDP families. Preferred members of the AP-1 family are c-Fos and c-Jun.

cFOS is a human proto-oncogene that belongs to the FOS family of transcription factors and encodes a 62 kDa protein, which forms a heterodimer with c-jun (part of Jun family of transcription factors), resulting in the formation of AP-1 (Activator Protein-1) complex which binds DNA at AP-1 specific sites at the promoter and enhancer regions of target genes and converts extracellular signals into changes of gene expression.

The CREB (cAMP-responsive element-binding protein) family is as family of transcription factors that binds cAMP response elements (CRE) containing the highly conserved nucleotide sequence, 5'-TGACGTCA-3', thereby modulating target gene expression. Preferred members of the CREB family are CREB1 and ATF4, more preferably human CREB1 and ATF4.

In one embodiment the promoter which is responsive to carbohydrate metabolism is a calcium-responsive promoter, wherein the calcium responsive promoter is a synthetic promoter consisting of one or multiple tandem repeats of binding sites of transcription factors selected from the group consisting of the NFAT family, the NFkB family, the AP-1 family, and/or the CREB family and/or cFOS operably linked to one or multiple promoters wherein the one or multiple promoters do not comprise enhancer elements, i.e. is not a constitutive promoter.

In one embodiment the promoter which is responsive to carbohydrate metabolism is a calcium-responsive promoter, wherein the calcium responsive promoter is a synthetic promoter consisting of one or multiple tandem repeats of binding sites of NFAT operably linked to one or multiple promoters wherein the one or multiple promoters do not comprise enhancer elements, wherein the sequence of the NFAT-binding sites of the calcium responsive promoter contains one or multiple tandem repeats of the binding site of a mammalian cytokine promoter selected from the group consisting of interleukin (IL)-2, IL-3, IL-4 promoter, peroxisome- proliferator-activated receptor-γ (PPARγ) promoter, orphan nuclear receptor 77 (NUR77) promoter, interferon γ promoter, GATA-binding protein 3 (GATA3) promoter and promoters of the T-box family of transcription factors (T-bex and eomesodermin), preferably the interleukin (IL)-2, IL-4 promoter and PPARγ promoter and most preferrably the murine interleukin (IL)-4 promoter in particular one or multiple tandem repeats of the binding site of the murine interleukin (IL)-4 promoter as shown in SEQ ID NO: 34, preferably 3 to 9 tandem repeats, more preferably 3, 5, 7 or 9 tandem repeats..

In one embodiment the promoter which is responsive to carbohydrate metabolism comprises SEQ ID NO: 4

In one embodiment the promoter which is responsive to carbohydrate metabolism comprises SEQ ID NO: 5

In one embodiment the promoter which is responsive to carbohydrate metabolism comprises SEQ ID NO: 6

In one embodiment the promoter which is responsive to carbohydrate metabolism comprises SEQ ID NO: 39

In one embodiment the recombinant cell further comprises a nucleic acid construct coding for a cellular component for sensing extracellular carbohydrates, in particular coding for a cellular component for sensing extracellular glucose. Preferably the cellular component for sensing extracellular carbohydrates e.g. extracellular glucose is a membrane protein or a fragment thereof or a subunit of a membrane protein or a fragment thereof, more preferably a membrane protein or a fragment or subunit of a membrane protein or a fragment thereof selected from the group consisting of G-protein coupled receptors, SLC2A family glucose transporters, SLC5A family sodium-glucose linked transporters, potassium channels, calcium channels and sodium channels, most preferably a membrane protein or a fragment or subunit of a membrane protein or a fragment thereof selected from the group consisting of potassium channels, calcium channels and sodium channels, in particular calcium channels, more particular voltage-gated calcium channels.

In one embodiment the recombinant cell comprises a nucleic acid construct comprising a promoter which is responsive to carbohydrate metabolism of said recombinant cell, wherein the promoter is operably linked to a gene coding for a therapeutic protein and further comprises a nucleic acid construct coding for a cellular component for sensing extracellular carbohydrates.

In one embodiment the recombinant cell further comprises a nucleic acid construct coding for a cellular component for sensing extracellular carbohydrates e.g. extracellular glucose, wherein the cellular component for sensing extracellular carbohydrates e.g. extracellular glucose is a voltage-gated calcium channel, preferably a subunit of a voltage-gated calcium channel, preferably a α1 subunit of a voltage-gated calcium channel selected from the group consisting of Caᵥ1.1, Caᵥ1.2, Caᵥ1.3, Caᵥ1.4, Caᵥ2.1, Caᵥ.2.2, Caᵥ2.3, Caᵥ3.1, Caᵥ3.2 and Caᵥ3.3, more preferably Caᵥ1.2, Caᵥ1.3, Caᵥ2.2, and most preferably Caᵥ1.3.

In one embodiment the recombinant cell is a cell which express an autologous cellular component (i.e. a cell which autologously express) a cellular component for sensing extracellular carbohydrates, wherein the autologous cellular component for sensing extracellular carbohydrates is a membrane protein selected from the group consisting of G-protein coupled receptors, the SLC2A family glucose transporters, the SLC5A family sodium-glucose linked transporters, potassium channels, calcium channels and sodium channels, in particular calcium channels, more particular voltage-gated calcium channels. In a preferred embodiment, the recombinant cell autologously express a calcium channel, preferably a voltage-gated calcium channel, more preferably a subunit of a voltage-gated calcium channel, most preferably a α1 subunit of a voltage-gated calcium channel selected from the group consisting of Caᵥ1.1, Caᵥ1.2, Caᵥ1.3, Caᵥ1.4, Caᵥ2.1, Caᵥ2.2, Caᵥ2.3, Caᵥ3.1, Caᵥ3.2 and Caᵥ3.3, more preferably Caᵥ1.2, Caᵥ1.3, Caᵥ2.2, and most preferably Caᵥ1.3.

Usually the cellular component for sensing extracellular carbohydrates of the recombinant cell of the present invention activates the promoter which is responsive to carbohydrate metabolism comprised by said recombinant cell e.g. the cellular component for sensing extracellular carbohydrates of the recombinant cell of the present invention activates the promoter which is responsive to carbohydrate metabolism comprised by said recombinant cell, wherein the gene coding for a therapeutic protein to which the promoter is operably linked is expressed, wherein the expression level of said gene correlates with the levels of extracellular carbohydrates. In a particular embodiment the cellular component for sensing extracellular carbohydrates of the recombinant cell of the present invention activates the promoter via calcium influx into the recombinant cell.

In one embodiment the recombinant cell is a non-pancreatic cell, preferably a non-pancreatic mammalian cell, more preferably a non-pancreatic human cell.

In one embodiment the recombinant cell is a mammalian cell, preferably a human cell. Recombinant mammalian cells are preferably mammalian cells selected from the group consisting of kidney cells, liver cells, stem cells, blood cells, brain cells, nerve cells, intestinal cells, fibroblasts, and adipose-derived cells, more preferably stem cells, kidney cells or liver cells, most preferably stem cells or kidney cells, in particular kidney cells. Recombinant human cells are preferably human cells selected from the group consisting of HEK-293, HeLa, mesenchymal stem cells (MSC), induced pluripotent stem cells (iPSC), pheochromocytoma of the rat adrenal medulla (PC12), the mouse neuroblastoma cell line (N2A), liver hepatocellular carcinoma (HepG2), enteroendocrine L-cells, human epithelial colorectal adenocarcinoma (Caco2), and clinical-grade human neural stem cell (CTX), preferably HEK-293, HeLa and MSC cells, most preferably HEK-293 cells.

In a specific embodiment, the recombinant cell of the present invention comprises a nucleic acid construct comprising a promoter which is responsive to glucose metabolism of said cell, wherein the promoter is operably linked to a gene coding for a therapeutic protein and optionally the recombinant cell further comprises a nucleic acid construct coding for a cellular component for sensing extracellular glucose.

In another specific embodiment, the recombinant cell of the present invention comprises a nucleic acid construct comprising a calcium-responsive promoter, wherein the promoter is operably linked to a gene coding for a therapeutic protein and optionally the recombinant cell further comprises a nucleic acid construct coding for a membrane protein or a fragment thereof or a subunit of a membrane protein or a fragment thereof, wherein the membrane protein is selected from the group consisting of G-protein coupled receptors, SLC2A family glucose transporters, SLC5A family sodium-glucose linked transporters, potassium channels, calcium channels and sodium channels, in particular calcium channels.

In another specific embodiment, the recombinant cell of the present invention comprises a nucleic acid construct comprising a calcium-responsive promoter, wherein the promoter is operably linked to a gene coding for a therapeutic protein and optionally the recombinant cell further comprises a nucleic acid construct coding for a voltage-gated calcium channel.

A particular, exemplary embodiment of the expression system of the invention is shown in Figure 2B. Herein, HEK-293 cells comprising a nucleic acid construct comprising a synthetic promoter comprising multiple tandem repeats of binding sites of a NFAT transcription factor and a nucleic acid construct coding for a Caᵥ1.3 channel are displayed, whereas extracellular glucose are taken up by the cells to generate increased amounts of ATP. The subsequent closure of ATP-sensitive potassium channels (K_{ATP}) activates Caᵥ1.3, resulting in increased Ca²⁺ influx and the calcium-dependent activation of NFAT-regulated transcription units.

In one embodiment the therapeutic protein is an insulinogenic agent selected from the group consisting of GLP1R-agonists, insulin, insulin analogues, growth hormones, insulin-like growth factors; an anorexic hormone; or a protein that activates brown fat metabolism, preferably selected from the group consisting of GLP1R-agonists, insulin and insulin analogues.

A GLP1R-agonist is any molecule that activates the GLP-1 receptor (GLP1R). GLP1R-agonists are usually selected from the group consisting of GLP-1, shGLP1, preferably shGLP1 as shown in SEQ ID NO: 35, exedin-4, exenatide, liraglutide, lixisenatide, albiglutide and dulaglutide. Preferred GLP1R-agonists are selected from the group consisting of shGLP1, preferably shGLP1 as shown in SEQ ID NO: 35 and exedin-4. Most preferred is shGLP1 or shGLP1 as shown in SEQ ID NO: 35.

Insulin analogues are usually selected from the group consisting of compounds derived from insulin that has been altered in its structure for the primary purpose of enhanced pharmaceutics or pharmacology. Preferred insulin analogues are selected from the group consisting of human, rodent, porcine, or bovine insulin, as well as Lispro, Aspart, Glulisine, Glargine and Detemir.

Growth hormones are usually selected from the group of hormones which stimulates growth in animal or plant cells, especially (in animals) that secreted by the pituitary gland. Preferred growth hormones are selected from the group consisting of ephinephrine, norepheniphrine and glucocorticoids.

Insulin-like growth factors are usually selected from the group consisting of hormones that are similar in molecular structure to insulin. Preferred insulin-like growth factors are selected from the group consisting of IGF1, IGF2 and IGFBP-6.

Anorexic hormones are usually selected from the group consisting of adiponectin, amylin, calcitonin, cholecystokinin (CCK), gastrin, gastric inhibitory polypeptide (GIP), ghrelin, leptin, motilin, pramlintide, secretin, somatostatin and peptide YY. Preferred anorexic hormones are selected from the group consisting of amylin, adiponectin, amylin- and adiponectin-analogues. Amylin- and adiponectin-analogues have usually an amino acid sequence identity of at least 70%, preferably at least 80%, more preferably at least 90%, most preferably at least 95%, in particular at least 97%, more particular at least 99% with the naturally occuring amylin and adiponectin, preferably with the naturally occuring human amylin and human adiponectin.

A protein that activates brown fat metabolism is usually selected from the group consisting of β2 AR activators, BMP7 irisin107, fibroblast growth factor 21 and natriuretic peptides. Preferred proteins that activates brown fat metabolism is selected from the group consisting of β2 AR activators and natriuretic peptides.

In a preferred embodiment the therapeutic protein is human insulin, human GLP-1 or a modified or truncated GLP-1, preferably the modified GLP1R-agonist shGLP1as shown in SEQ ID NO: 35.

In one embodiment the therapeutic protein is an agent against a metabolic disease, wherein the metabolic disease is selected from the group consisting of T1D (type-1 diabetes), T2D (type-2 diabetes), diabetic ketoacidosis, obesity, cardiovascular disease, the metabolic syndrome and cancer. Preferably the metabolic disease is selected from the group consisting of T1D (type-1 diabetes), T2D (type-2 diabetes), diabetic ketoacidosis, and the metabolic syndrome.

Type 1 diabetes (also known as diabetes mellitus type 1) is a form of diabetes mellitus that results from the autoimmune destruction of the insulin-producing beta cells in the pancreas. The subsequent lack of insulin leads to increased glucose in blood and urine.

Type 2 diabetes (also known as diabetes mellitus type 2) is a long term metabolic disorder that is characterized by high blood sugar, insulin resistance, and relative lack of insulin.

Cardiovascular diseases are usually selected from the group consisting of consisting of: myocardial interstitial disease, cardiac fibrosis, heart failure such as heart failure with diastolic heart failure (DHF), heart failure with preserved ejection fraction (HFpEF), congestive heart failure (CHF), asymptomatic left ventricular diastolic dysfunction (ALVDD), coronary atherosclerosis, cancer and diabetes, inflammatory bowel disease, chronic prostatitis, infections, pulmonary inflammation, osteomyelitis, renal disease, gout, arthritis and shock.

Metabolic syndrome (also referred to as syndrome X) is a cluster of risk factors that is responsible for increased cardiovascular morbidity and mortality. The National Cholesterol Education Program - Adult Treatment panel (NECP - ATP III) identified metabolic syndrome as an independent risk factor for cardiovascular disease. (National Institutes of Health: Third Report of the National Cholesterol Education Program Expert Panel on Detection, Evaluation, and Treatment of High Blood Cholesterol in Adults (Adult Treatment Panel III). Executive publication no. 01-3670). As used herein, metabolic syndrome is defined according to the World Health Organization criteria (1999) which require presence of diabetes mellitus, impaired glucose tolerance, impaired fasting glucose or insulin resistance, AND two of the following: blood pressure: ≥ 140/90 mmHg; dyslipidaemia: triglycerides (TG): ≥ 1.695 mmol/L and high-density lipoprotein cholesterol (HDL-C) < 0.9 mmol/L (male), < 1.0 mmol/L (female); central obesity: waist:hip ratio > 0.90 (male); > 0.85 (female), and/or body mass index > 30 kg/m2; microalbuminuria: urinary albumin excretion ratio ≥ 20 mg/min or albumin:creatinine ratio ≥ 30 mg/g.

In one specific embodiment the therapeutic protein is an agent against T1D and/or T2D.

In a further aspect the present invention provides an encapsulated cell, comprising the recombinant cell as described above and a semi-permeable membrane.

Encapsulated cell biodelivery is based on the concept of isolating cells from the recipient host's immune system by surrounding the cells with a semipermeable biocompatible membrane before implantation within the host. Cells are immunoisolated from the host by enclosing them within implantable polymeric capsules formed by a semi-permeable membrane. This approach prevents the cell-to-cell contact between host and implanted tissues, eliminating antigen recognition through direct presentation.

The encapsulated cell of the present invention has a semi-permeable membrane which is tailored to control diffusion of molecules, such as growth factor hormones, neurotransmitters, peptides, antibodies and complements, based on their molecular weight. Using encapsulation techniques, cells can be transplanted into a host without immune rejection, either with or without use of immunosuppressive drugs. Useful biocompatible polymer capsules usually contain a core that contains cells, either suspended in a liquid medium or immobilised within an immobilising matrix, and a surrounding or peripheral region of permselective matrix or membrane ("jacket") that does not contain isolated cells, that is biocompatible, and that is sufficient to protect cells in the core from detrimental immunological attack. Encapsulation hinders elements of the immune system from entering the capsule, thereby protecting the encapsulated cells from immune destruction. The semipermeable nature of the membrane also permits the biologically active molecule of interest to easily diffuse from the capsule into the surrounding host tissue and allows nutrients to diffuse easily into the capsule and support the encapsulated cells. The capsule can be made from a biocompatible material. A "biocompatible material" is a material that, after implantation in a host, does not elicit a detrimental host response sufficient to result in the rejection of the capsule or to render it inoperable, for example through degradation. The biocompatible material is relatively impermeable to large molecules, such as components of the host's immune system, but is permeable to small molecules, such as insulin, growth factors, and nutrients, while allowing metabolic waste to be removed. A variety of biocompatible materials are suitable for delivery of growth factors by the composition of the invention. Numerous biocompatible materials are known, having various outer surface morphologies and other mechanical and structural characteristics as described e.g. by WO 92/19195 or WO 95/05452. Components of the biocompatible material may include a surrounding semipermeable membrane and the internal cell-supporting scaffolding. Preferably, the recombinant cells are seeded onto the scaffolding, which is encapsulated by the permselective membrane. The filamentous cell-supporting scaffold may be made from any biocompatible material selected from the group consisting of acrylic, polyester, polyethylene, polypropylene polyacetonitrile, polyethylene teraphthalate, nylon, polyamides, polyurethanes, polybutester, silk, cotton, chitin, carbon, or biocompatible metals. Also, bonded fibre structures can be used for cell implantation (U.S. Pat. No. 5,512,600, incorporated by reference). Biodegradable polymers include those comprised of poly(lactic acid) PLA, poly(lactic-coglycolic acid) PLGA, and poly(glycolic acid) PGA and their equivalents. Foam scaffolds have been used to provide surfaces onto which transplanted cells may adhere (WO 98/05304, incorporated by reference). Woven mesh tubes have been used as vascular grafts (WO 99/52573, incorporated by reference). Additionally, the core can be composed of an immobilizing matrix formed from a hydrogel, which stabilizes the position of the cells. A hydrogel is a 3-dimensional network of cross-linked hydrophilic polymers in the form of a gel, substantially composed of water.

Various polymers and polymer blends can be used to manufacture the semipermeable membrane, including alginate, alginate-poly-(L-lysine)-alginate, polycarbonate, polyethylene, polyethylene-terephthalate, collagen, gelatin, agarose, cellulose acetate and cellulose sulfate, polyacrylates (including acrylic copolymers), polyvinylidenes, polyvinyl chloride copolymers, polyurethanes, polystyrenes, polyamides, cellulose acetates, cellulose nitrates, polysulfones (including polyether sulfones), polyphosphazenes, polyacrylonitriles, poly(acrylonitrile/covinyl chloride), as well as derivatives, copolymers and mixtures thereof. Such membranes, and methods of making them are disclosed by e.g. U.S. Pat. Nos. 5,284,761 and 5,158,881.

The capsule can be any configuration appropriate for maintaining biological activity and providing access for delivery of the product or function, including for example, cylindrical, rectangular, disk-shaped, patch-shaped, ovoid, stellate, or spherical. Moreover, the capsule can be coiled or wrapped into a mesh-like or nested structure. If the capsule is to be retrieved after it is implanted, configurations, which tend to lead to migration of the capsules from the site of implantation, such as spherical capsules small enough to travel in the recipient host's blood vessels, are not preferred. Certain shapes, such as rectangles, patches, disks, cylinders, and flat sheets offer greater structural integrity and are preferable where retrieval is desired.

The encapsulated cell devices are implanted according to known techniques. Many implantation sites are contemplated for the devices and methods of this invention. These implantation sites include, but are not limited to the intraperitoneal cavity, into the kidney capsules, subcutaneous tissues, the portal vein, the liver and the cerebral cortex.

In one embodiment the encapsulated cell comprises a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In one embodiment the semi-permeable membrane comprises a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In a further aspect the present invention provides thus a method for producing a therapeutic protein in vivo in a mammal, said method comprising:
(a) providing an in vitro population of the recombinant cells as described herein into an implantable semi-permeable device;
(b) implanting the device with the cell population into a mammalian host; and
(c) maturing the cell population in said device in vivo such that at least some cells of the cell population are cells that produce a therapeutic protein in response to carbohydrate stimulation in vivo.

In one embodiment the implantable semi-permeable device are capsules or beads consisting of a biocompatible material selected from the group consisting of alginate, alginate-poly-(L-lysine)-alginate, polycarbonate, polyethylene, polyethylene-terephthalate, collagen, gelatin, agarose, cellulose acetate and cellulose sulfate, preferably alginate, more preferably alginate-poly-(L-lysine)-alginate.

In a further aspect the present invention provides an in vitro cell culture comprising the recombinant cell as described herein, wherein said recombinant cell is expressing a therapeutic protein in the presence of carbohydrates, preferably in the presence of glucose. Cells can be grown and maintained in vitro as generally known. The nutrient medium and the cells generally are contained in a suitable vessel to which an adequate supply of oxygen and carbon dioxide is furnished in order to support cell growth and maintenance. Cell cultures may be batch systems in which nutrients are not replenished during cultivation but oxygen is added as required, fed-batch systems in which both nutrient and oxygen concentrations are monitored and replenished as necessary, and perfusion systems in which nutrient and waste product concentrations are monitored and controlled. Cells may be cultured in a variety of media. Commercially available media such as Ham's F10 (Sigma-Aldrich Chemie GmbH, Buchs, Switzerland), Minimal Essential Medium (MEM; Sigma-Aldrich Chemie GmbH), RPMI-1640 (Sigma-Aldrich Chemie GmbH, Basel, Switzerland), and Dulbecco's Modified Eagle's Medium ((DMEM; Sigma-Aldrich Chemie GmbH) are suitable for culturing the host cells.

In a further aspect the present invention provides a method, preferably an in vitro method, of producing a recombinant cell expressing a therapeutic protein, said method comprising the steps of:
(a) obtaining a population of cells;
(b) transfecting said population of cells with a nucleic acid construct comprising a promoter which is responsive to a product of the carbohydrate metabolism of said cell, wherein the promoter is operably linked to a gene coding for a therapeutic protein;
(c) incubating the population of transfected cell in the presence of carbohydrates for a sufficient time to permit the transfected cells to express a therapeutic protein.

In one embodiment the population of cells is further transfected in step (b) with a nucleic acid construct encoding a cellular component for sensing extracellular carbohydrates. The cells, the promoter which is responsive to a product of the carbohydrates metabolism, and the cellular component for sensing extracellular carbohydrates used in the method are as described above. The carbohydrate used is preferably glucose. Incubation time in the presence of carbohydrates is usually between 0.5 to 96 hours, preferably between 1 to 12 hours.

In a further aspect the present invention provides the recombinant cell or the encapsulated cell as described herein for use as a medicament.

In a further aspect the present invention provides the recombinant cell or the encapsulated cell as described herein for use in a method for the prevention, delay of progression or treatment of a metabolic disease in a subject.

Also provided is the use of the recombinant cell or the encapsulated cell as described herein for the manufacture of a medicament for the prevention, delay of progression or treatment of a metabolic disease in a subject.

Also provided is the use of the recombinant cell or the encapsulated cell as described herein for the prevention, delay of progression or treatment of a metabolic disease in a subject.

Also provided is a method for the prevention, delay of progression or treatment of a metabolic disease in a subject, comprising administering to said subject the recombinant cell or the encapsulated cell as described herein.

In one embodiment the metabolic disease is selected from the group consisting of T1D (type-1 diabetes), T2D (type-2 diabetes), diabetic ketoacidosis, obesity, cardiovascular disease, the metabolic syndrome and cancer. Preferably the metabolic disease is selected from the group consisting of T1D (type-1 diabetes), T2D (type-2 diabetes), diabetic ketoacidosis, and the metabolic syndrome.

In a further aspect the present invention provides a method to deliver a nucleic acid construct to a cell, wherein the nucleic acid construct comprises a promoter which is responsive to carbohydrate metabolism of said cell, wherein the promoter is operably linked to a gene coding for a therapeutic protein, comprising administering said nucleic acid construct to said cell, whereby said gene coding for a therapeutic protein is expressed in said cell in response to carbohydrate stimulation. In one embodiment the cell is further transfected with a nucleic acid construct encoding a cellular component for sensing extracellular carbohydrates. The promoter which is responsive to carbohydrate metabolism, and the cellular component for sensing extracellular carbohydrates used in the method are as described above. The carbohydrate used is preferably glucose.

In one embodiment the nucleic acid construct is delivered to a cell in a subject e.g. by gene therapy. Thus in a specific embodiment the present invention provides a method to deliver a nucleic acid construct to a cell of a subject, wherein the nucleic acid construct comprises a promoter which is responsive to carbohydrate metabolism of said cell, wherein the promoter is operably linked to a gene coding for a therapeutic protein, comprising administering said nucleic acid construct to said subject, whereby said gene coding for a therapeutic protein is expressed in said cell of said subject in response to carbohydrate stimulation.

For delivery of a nucleic acid construct to a cell in a subject it may be valuable in some instances to utilize or design vectors to deliver the nucleic acid construct to a particular cell type. Certain vectors exhibit a natural tropism for certain tissue types. Cell type specificity or cell type targeting may be achieved in vectors derived from viruses having characteristically broad infectivities by the modification of the viral envelope proteins. For example, cell targeting has been achieved with adenovirus vectors by selective modification of the viral genome knob and fiber coding sequences to achieve expression of modified knob and fiber domains having specific interaction with unique cell surface receptors.. Other methods of cell specific targeting have been achieved by the conjugation of antibodies or antibody fragments to the envelope proteins. Alternatively, particularly moieties may be conjugated to the viral surface to achieve targeting. Additionally, the virally encoded nucleic acid construct may also be under control of a tissue specific promoter region allowing expression of the gene coding for a therapeutic protein preferentially in particular cell types.

It will be apparent to one skilled in the art that nucleic acid constructs according to the invention may be introduced into an animal subject in a variety of ways including enterally (orally, rectally or sublingually) or parenterally (intravenously, subcutaneously, or by inhalation). The nucleic acid constructs may be provided to the mammal by e.g. implanted catheters. The nucleic acid constructs can be instilled into a body cavity to facilitate transduction of the surrounding tissues. Examples of such body cavities into which the solutions may be provided for the delivery of nucleic acids include the peritoneal cavity, pleural cavity, and the abdominal cavity. Additionally the nucleic acid constructs may be provided in other fluid containing spaces.

The nucleic acid constructs to be delivered to a cell of a subject may further comprise additional carriers, excipients or diluants. The compositions comprising the nucleic acid constructs may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. The concentration of the nucleic acid constructs in the compositions can vary widely, i.e., from less than about 0.1%, usually at or at least about 2% to as much as 20% to 50% or more by weight, and will be selected primarily by fluid volumes, viscosities, etc., in accordance with the particular mode of administration selected.

### EXAMPLES

### General experimental procedures

***Vector** Design.* Comprehensive design and construction details for all expression vectors are provided in **Table 1.** Plasmids encoding K_{ATP}-subunits (pCMV Human SUR1 and pCMV6c hKir6.2(BIR)) were kindly provided by Susumu Seino (Kobe University, Kobe, Japan). Plasmids encoding Caᵥ2.2-, Caᵥ1.2- and Caᵥ1.3-subunits (CaV1.3e[8a,11,31b,Δ32,42a], CaV1.2, Cav2.2e[Δa10, Δ18a, Δ24a, 31a, 37a, 46], Cavb3 and CaVα2δ1) were kindly provided by Diane Lipscombe (Brown University, RI, USA).

### Table 1: Plasmids and oligonucleotides used and designed herein

Oligonucleotides: restriction endonuclease-specific sites are underlined in oligonucleotide sequences. Annealing base pairs contained in oligonucleotide sequences are shown in capital letters.

**Abbreviations: *att*R1/2,** Gateway®-compatible recombination sites; **Caᵥ1.2,** member 2 of the Caᵥ1 family of L-type voltage-gated Ca²⁺ channels; **Caᵥ1.3**, member 3 of the Caᵥ1 family of L-type voltage-gated Ca²⁺ channels; **Caᵥ2.2**, N-type voltage-gated Ca²⁺ channel; **c-fos,** human proto-oncogene from the Fos family of transcription factors**;Cacna1b**, α1-subunit of rat Caᵥ2.2 (NCBI Gene ID: 257648); **Cacnalc,** α1-subunit of mouse Caᵥ1.2 (NCBI Gene ID: 12288); **Cacnald,** α1-subunit of rat Caᵥ1.3 (NCBI Gene ID: 29716); **Cacnb3,** β3-subunit of rat Caᵥ1.3 (NCBI Gene ID: 25297); **Cacna2d1,** α2δ-subunit of rat Caᵥ1.3 (NCBI Gene ID: 25399); ***ccd*B**, DNA gyrase toxin for positive selection; *Cmr,* chloramphenicol-resistance gene for negative selection; **CRE,** cAMP-response element; **GLuc,** *Gaussia princeps* luciferase; **hGCK,** human pancreas glucokinase (also known as hexokinase 4; NCBI Gene ID: 2645); **hGlut2**, human facilitated glucose transporter member 2 (also known as SLC2A2; NCBI Gene ID: 6514); **hGNAT3**, human guanine nucleotide binding protein alpha transducing 3 (NCBI Gene ID: 346562); **hKir6.2,** human inwardly rectifying KATP-channel subunit (also known as KCNJ11; NCBI Gene ID: 3767); **hSUR1**, regulatory sulphonylurea receptor subunit of human K_{ATP}-channels (also known as ABCC8; NCBI Gene ID: 6833); **hT1R2,** member 2 of the human taste receptor type 1 family (NCBI Gene ID: 80834); **hT1R3,** member 3 of the human taste receptor type 1 family (NCBI Gene ID: 83756); **IL2/4,** interleukin 2/4; **K_{ATP}**, adenosine triphosphate-sensitive potassium channel; **MCS,** multiple cloning site; **mINS**, modified rat insulin variant as shown in SEQ ID NO: 36 for optimal expression in HEK-293 cells; **NFAT**, nuclear factor of activated T-cells; **NF-κB**, nuclear factor kappa-light-chain-enhancer of activated B cells; **pA**, polyadenylation signal; **P**_{cFOS}, synthetic mammalian promoter containing a tetrameric c-fos response element ((c-fos)₄-Pₘᵢₙ; Sheng M et al., Mol Cell Biol 8, 2787-96 (1988)); **PCR**, polymerase chain reaction; **P_{CRE},** CRE-containing synthetic mammalian promoter; **P_{CREm}**, modified PCRE variant; **PEST,** peptide sequence rich in proline, glutamic acid, serine and threonine; **P_{hCMV}**, human cytomegalovirus immediate early promoter; **P**_{**hCMV***-1}, tetracycline-responsive promoter (tetO₇-P_{hCMVmin}); **P_{hCMVmin}**, minimal version of P_{hCMV}; **Pₘᵢₙ**, minimal eukaryotic TATA-box promoter (5'-TAGAGGGTATATAATGGAAGCTCGACTTCCAG-3') as shown in SEQ ID NO: 33; **P**_{**NFAT**-**IL2**}, synthetic mammalian promoter containing three tandem repeats of a murine IL2 NFAT-binding site ((NFAT_{IL2})₃-Pₘᵢₙ; Rooney JW et al., EMBO J 13, 625-633 (1994)); **P_{NFAT}-_{IL4}**, synthetic mammalian promoter containing three tandem repeats of a murine IL4 NFAT-binding site ((NFAT_{IL4})₃-Pₘᵢₙ; Rooney JW et al., EMBO J 13, 625-633 (1994)); **P**_{NFAT-IL4x5}, synthetic mammalian promoter containing five tandem repeats of a human IL4 NFAT-binding site ((NFAT_{IL4})₅-Pₘᵢₙ); **P_{NFAT-IL4x7}**, synthetic mammalian promoter containing seven tandem repeats of a human IL4 NFAT-binding site ((NFAT_{IL4})₇-Pₘᵢₙ); **P_{NFAT-IL4x9},** synthetic mammalian promoter containing nine tandem repeats of a human IL4 NFATbinding site ((NFAT_{IL4})₉-Pₘᵢₙ); **P_{NFkB}**, synthetic mammalian promoter containing a NF-κB-response element (Genbank: EU581860.1); **P_{SV40}**, simian virus 40 promoter; **SEAP**, human placental secreted alkaline phosphatase; **shGLP1**, short human glucagon-like peptide 1; ***tetO***, TetR-specific operator; **TetR,** *Escherichia coli Tn* 10-derived tetracycline-dependent repressor of the tetracycline resistance gene; **TurboGFP:dest1,** PEST-tagged TurboGFP variant (Evrogen); *zeo*, gene conferring zeocin resistance. **Oligonucleotides:** Restriction endonuclease-specific sites are underlined and annealing base pairs are indicated in capital letters.

| **Plasmid** | **Description and Cloning Strategy** | **Reference or Source** |
|---|---|---|
| pCaV1.2 | pcDNA3.1(+)-derived constitutive Cacna1c expression vector (P_{hCMV}-Cacna1c-pA) (Addgene no. 26572). | Helton et al., 2005 |
| pCaV1.3 | pcDNA3.1(+)-derived constitutive Cacna1d expression vector (P_{hCMV}-Cacna1d-pA) (Addgene no. 26576). | Xu and Lipscombe, 2001 |
| pCavb3 | pcDNA3.1(+)-derived constitutive Cacnb3 expression vector (P_{hCMV}-Cacnb3-pA) (Addgene no. 26574). | Prof. Lipscombe Lab |
| pCaVα2δ1 | pcDNA3.1(+)-derived constitutive Cacna2d1 expression vector (P_{hCMV}-Cacna2d1-pA) (Addgene no. 26575). | Lin et al., 2004 |
| pCav2.2 | pcDNA3.1(+)-derived constitutive Cacna1b expression vector (P_{hCMV}-Cacna1b-pA) (Addgene no. 26569). | Bell et al., 2004 |
| pcDNA3.1(+ ) | Mammalian expression vector (P_{hCMV}-MCS-pA). | Life Technologies, CA |
| pcDNA3.2/v 5-DEST | Mammalian Gateway^{®}-compatible destination vector (P_{hCMV}-*att*R1*-Cm^{r}-ccd*B*-att*R2*-*pA)*.* | Life Technologies, CA |
| pcDNA3.2/v 5-DEST-hGlut2 | pcDNA3.2/v5-DEST containing a constitutive hGlut2 expression unit (P_{hCMV}-hGlut2-pA) (Addgene no. 18086). | Takanaga and Frommer, 2010 |
| pCMV6 | Mammalian expression vector (P_{hCMV}-MCS-pA). | Yamada et al., 1992 |
| pCMV6-hSUR1 | pCMV6 containing a constitutive hSUR1 expression unit (P_{hCMV}-hSUR1-pA). | Beguin et al., 1999 |
| pCMV6-hKir6.2 | pCMV6 containing a constitutive hKir6.2 expression unit (P_{hCMV}-hKir6.2-pA). | Inagaki et al., 1995 |
| pDA43 | Tetracycline-responsive GLuc expression vector (P_{hCMV*-1}-GLuc-pA). | Muller et al., 2012 |
| pDA145 | P_{CRE}-driven mINS-expression vector (P_{CRE}-mINS-pA). | Auslander et al., 2014 |
| pDONR | Gateway^{®}-compatible cloning vector. | Life Technologies, CA |
| pDONR-hGCK | pDONR containing hGCK (Addgene no. 23750). | Johannessen et al., 2010 |
| pGNAT3 | pCMV6 containing a constitutive hGNAT3 expression unit (P_{hCMV}-GNAT3-pA). | OriGene, MD |
| pHY30 | P_{NFAT-IL2}-driven SEAP expression vector ((NFAT_{IL2})₃-Pₘᵢₙ-SEAP-pA). | Ye et al., 2011 |
| pHY57 | P_{NFAT-IL2}-driven shGLP1 expression vector ((NFAT_{IL2})₃-Pₘᵢₙ-shGLP1-pA). | Ye et al., 2011 |
| pSP16 | P_{CREm}-driven SEAP expression vector (P_{CREm}-SEAP-pA). | Saxena et al., 2016 |
| pUC57 | pUC19-derived prokaryotic expression vector. | GeneScript, NJ |
| pZeoSV2(+) | Constitutive mammalian expression vector conferring zeocin resistance (P_{hCMV}-*zeo*-pA). | Life Technologies, CA |
| pFS119 | P_{NFAT-IL4x5}-driven TurboGFP:dest1 expression vector ((NFAT_{IL4})₅-Pₘᵢₙ-TurboGFP:dest1-pA). | Sedlmayer of Prof. Fussenegger's lab |
| | TurboGFP:dest1 was PCR-amplified using oligonucleotides oFS200 (5'-tgttggtaaagaattcgcccaccaagctttaagccaccATGGAGAGCGACG AGAGCGGCC-3') and oMX78 (5'-ctttaaaaaacctcccacacctccc-3'), restricted with *Eco*RI*lHpa*I and cloned into the corresponding sites (*Eco*RI*lHpa*I*)* of pMX57. | |
| pKR32 | P_{NFkB}-driven SEAP expression vector (P_{NFkB}-SEAP-pA). Custom-designed P_{NFkB} was restricted with *Mlu*I/*Hind*III and cloned into the corresponding sites (*Mlu*I/*HindIII*) of pHY30. | Rössger of Prof. Fussenegger's lab |
| pT1R2 | Constitutive hT1R2 expression vector (P_{hCMV}-hT1R2-pA). | Wieland of Prof. |
| | Custom-designed hT1R2 was restricted with *Eco*RI/*Xba*I and cloned into the corresponding sites (*Eco*RI*lXba*I) of pcDNA3.1(+). | Fussenegger's lab |
| pT1R3 | Constitutive hT1R3 expression vector (P_{hCMV}-hT1R3-pA). | Wieland of Prof. |
| | Custom-designed hT1R3 was restricted with *Eco*RI*lXba*I and cloned into the corresponding sites (*Eco*RI/*Xba*I) of pcDNA3.1(+). | Fussenegger's lab |
| pMX53 | P_{cFOS}-driven SEAP expression vector (P_{cFOs}-SEAP-pA; P_{cFOS}, (c-fos)₄-Pₘᵢₙ). | This work |
| | Pₘᵢₙ-SEAP was PCR-amplified from pSP16 using oligonucleotides OMX59 (5'-cgcgtgctagcagcctgacgtttcagagactgacgtttcagagactgacgtttcagaga ctgacgtttcagatctctcga ggtcgacagcggAGACTCTAGAGGGTATATAATG-3') and OMX24 (5'-CTTGAGCACATAGCCTGGACCGTTTCCGTA-3'), restricted with *Nde*I*lNhe*I and cloned into the corresponding sites (*Nde*I*lNhe*I) of pHY30. | |
| pMX56 | P_{NFAT-IL4}-driven SEAP expression vector ((NFAT_{IL4})₃-Pₘᵢₙ-SEAP-pA). | This work |
| | Pₘᵢₙ-SEAP was PCR-amplified from pSP16 using oligonucleotides OMX63 (5'- cgcgtg-[ctagctacattggaaaattttatacacgtt]₃AGACTCTAGAGGGTATAT AA-3') and OMX24 (5'-CTTGAGCACATAGCCTGGACCGTTTCCGTA-3'), restricted with *Nde*I*lNhe*I and cloned into the corresponding sites (*Nde*I*lNhe*I*)* of pHY30. | |
| pMX57 | P_{NFAT-IL4x5}-driven SEAP expression vector ((NFAT_{IL4})₅-Pₘᵢₙ-SEAP-pA). | This work |
| | P_{NFAT-IL4x5} (5'-cgcgtg-[ctagctacattggaaaattttatacacgtt]5-agactc-Pₘᵢₙ-cttggcaatccggtactgttggtaaagaattcgcccacc-3') was excised from pMX570 with *Nhe*I*lEco*RI and cloned into the corresponding sites (*Nhe*I/*EcoR*I) of pMX56. | |
| pMX58 | P_{NFAT-IL4x7}-driven SEAP expression vector ((NFAT_{IL4})₇-Pₘᵢₙ-SEAP-pA). | This work |
| | P_{NFAT-ILAx7} (5'- cgcgtg-[ctagctacattggaaaattttatacacgtt]₇-agactc-Pₘᵢₙ-cttggcaatccggtactgttggtaaagaattcgcccacc-3') was excised from pMX580 with *Nhe*I*lEco*RI and cloned into the corresponding sites (*Nhe*I/*EcoR*I) of pMX56. | |
| pMX59 | P_{NFAT-IL4x9}-driven SEAP expression vector ((NFAT_{IL4})₉-Pₘᵢₙ-SEAP-pA). | This work |
| | P_{NFAT-IL4x9} (5'- cgcgtg-[ctagctacattggaaaattttatacacgtt]₉-agactc-Pₘᵢₙ-cttggcaatccggtactgttggtaaagaattcgcccacc-3') was excised from pMX590 with *Nhe*I*lEco*RI and cloned into the corresponding sites (*Nhe*I/*EcoR*I) of pMX56. | |
| pMX61 | P_{NFAT-IL4x5}-driven shGLP1-expression vector ((NFAT_{IL4})₅-Pₘᵢₙ-shGLP1-pA). | This work |
| | shGLP1 was PCR-amplified from pHY57 using oligonucleotides OMX70 (5'-ctgttggtaaagaattcgcccaccATGAAGATCATCCTGTGGCTGT GTG-3') and OMX71 (5'-ggagtcgacgcgtGAAGCGGCCGGCCTCATTTACCAGGAGA GTGGG-3' restricted with *Eco*RI/*Fse*I and cloned into the corresponding sites (*Eco*RI*lFse*I) of pMX57. | |
| pMX68 | P_{NFAT-IL4x5}-driven mINS-expression vector ((NFAT_{IL4})₅-Pₘᵢₙ-mINS-pA). | This work |
| | mINS was PCR-amplified from pDA145 using oligonucleotides OMX72 (5'-ctgttggtaaagaattcgcCCACCATGGCCCTGTGGATGCGCTT CCTGC-3') and OMX73 (5'-CTGAAACATAAAATGAATGCAATTGTTGTTG-3'), restricted with *Eco*RI*lMfe*I and cloned into the corresponding sites (*Eco*RI*lMfe*I) of pMX57. | |
| pMX90 | Constitutive hGCK expression vector (P_{hCMV}-hGCK-pA). | This work |
| | GCK was PCR-amplified from pDONR-hGCK using oligonucleotides OMX100 (5'-ctggaattccaccATGCTGGACGACAGAGCCAGG-3') and OMX101 (5'-ctctagatgcatgctcgagTCACTGGCCCAGCATACAGGCCTTC -3' restricted with *Eco*RI*lXho*I and cloned into the corresponding sites (*Eco*RI*lXho*I) of pcDNA3.1(+). | |
| pMX99 | P_{NFAT-IL4x7}-driven mINS-expression vector ((NFAT_{IL4})₇-Pₘᵢₙ-mINS-pA). | This work |
| | mINS was excised from pMX68 using *Eco*RI/*Hpa*I and cloned into the corresponding sites (*Eco*RI*lHpa*I*)* of pMX58. | |
| pMX100 | P_{NFAT-IL4x9}-driven mINS-expression vector ((NFAT_{IL4})₉-Pₘᵢₙ-mINS-pA). | This work |
| | mINS was excised from pMX68 using *Eco*RI*lHpa*I and cloned into the corresponding sites (*Eco*RI*lHpa*I*)* of pMX59. | |
| pMX115 | P_{NFAT}-_{IL4x9}-driven shGLP1-expression vector ((NFAT_{IL4})₉-Pₘᵢₙ-shGLP1-pA). | This work |
| | shGLP1 was excised from pMX61 using *Eco*RI*lHpa*I and cloned into the corresponding sites (*Eco*RI*lHpa*I*)* of pMX59. | |
| pMX117 | P_{NFAT-IL4x7}-driven shGLP1-expression vector ((NFAT_{IL4})₉-Pₘᵢₙ-shGLP1-pA). | This work |
| | shGLP1 was excised from pMX61 using *Eco*RI*lHpa*I and cloned into the corresponding sites (*Eco*RI/*Npa*I) of pMX58. | |
| pMX570 | pUC57-derived vector containing custom-designed P_{NFAT}-_{IL4x5}. | This work |
| pMX580 | pUC57-derived vector containing custom-designed P_{NFAT-IL4x7}. | This work |
| pMX590 | pUC57-derived vector containing custom-designed P_{NFAT-IL4x9}. | This work |
| pWH29 | P_{NFAT-IL4x5}-driven GLuc expression vector ((NFAT_{IL4})₅-Pₘᵢₙ-GLuc-pA). | This work |
| | P_{NFAT-ILAx5} was excised from pMX57 using *Mlu*I/*Eco*RI and cloned into the corresponding sites (*Mlu*I/*Eco*RI) of pDA43. | |

***Cell culture and transfection.*** Human embryonic kidney cells (HEK-293T, ATCC: CRL-11268) were cultured in Dulbecco's modified Eagle's medium (DMEM, Invitrogen) supplemented with 10% (v/v) fetal bovine serum (FBS; Sigma-Aldrich, Buchs, Switzerland; cat. no. F7524, lot no. 022M3395) and 1% (v/v) penicillin/streptomycin solution (PenStrep; Biowest, Nuaillé, France; cat. no. L0022-100) at 37°C in a humidified atmosphere containing 5% CO₂. For passaging, cells of pre-confluent cultures were detached by incubation in 0.05% Trypsin-EDTA (Life Technologies, CA, USA; cat. no. 25300-054) for 3min at 37°C, collected in 10ml cell culture medium, centrifuged for 3min at 290g and resuspended in fresh DMEM at standard cell densities (1.5 x 10⁵ cells / mL), before seeding into new tissue culture plates. Cell number and viability were quantified using an electric field multichannel cell counting device (Casy Cell Counter and Analyzer Model TT, Roche Diagnostics GmbH). For transfection, a solution containing 2.4µg plasmid DNA and 7.5µg polyethyleneimine (PEI; Polysciences, Eppelheim, Germany; cat. no. 24765-2) was incubated in 300µl serum- and antibiotics-free DMEM for 30min at 22°C and subsequently added dropwise to 3 x 10⁵ cells seeded per well of a 6-well plate. 12h after addition of PEI, transfected cells were detached by incubation in Trypsin-EDTA, centrifuged (3min at 290g) and resuspended in low/no-glucose medium (glucose-free DMEM [Life Technologies, CA, USA; cat. no. 11966-025] supplemented with 10% FBS, 1% PenStrep, 0-2mM D-glucose and 0.7mM CaCl₂) and reseeded at a cell density of 2 x 10⁵ / mL. Unless stated otherwise, D-glucose or other control compounds were added to transfected cells after cultivation under low/no-glucose conditions for another 12h.

***Quantification** of target gene expression.* Expression levels of human placental secreted alkaline phosphatase (SEAP) in culture supernatants were quantified according to *a p-*nitrophenylphosphate-based light absorbance time course (Wang H et al., Nucleic Acids Res 43(14):e91 (2015)). SEAP levels in mouse serum were profiled using a chemiluminescence-based assay (Roche Diagnostics GmbH, Mannheim, Germany; cat. no. 11 779 842 001). Murine insulin levels (mINS) in culture supernatants and mouse serum were quantified with a Mouse Insulin ELISA kit (Mercordia, Uppsala, Sweden; cat. no. 10-1247-01). Short human glucagon-like peptide 1 (shGLP1) levels in culture supernatants were quantified with a Mouse IgG ELISA Kit (Immunology Consultants Laboratory Inc., Portland, OR; cat. no. E-90G). Bioactive GLP-1 levels in mouse serum were quantified with a High Sensitivity GLP-1 Active ELISA Kit (Merck Millipore, Schaffhausen, Switzerland; cat. no. EZGLPHS-35K). TurboGFP was visualized by fluorescence microscopy using a Nikon Ti-E base Wide Field microscope (Nikon AG, Egg, Switzerland) equipped with a Hammamatsu Orca Flash 4 digital camera, a 20×objective, a 488 nm / 509 nm excitation and emission filter set and NIS Elements AR software (version 4.3.0).

***Generation of stable cell lines.*** The HEK-293_{NFAT-SEAP} cell line, transgenic for depolarization-stimulated SEAP expression, was constructed by co-transfecting HEK-293 cells with a 20:1 (w/w) mixture of pMX57 (P_{NFAT3}-SEAP-pA) and pZeoSV2(+) (P_{SV40}-*zeo*-pA), followed by selection in culture medium containing 1mg/mL zeocin (Life Technologies, CA, USA; cat. no. R250-05) and FACS-mediated single-cell cloning. Sixteen cell clones were picked and the best-in-class HEK-293_{NFAT-SEAP1} was used for all follow-up studies.

***Semi-quantitative RT-PCR.*** Total RNA of untreated HEK-293 cells was isolated using the ZR RNA MiniPrep™ kit (Zymo Research, CA, USA; cat. no. R1064), treated with DNaseI (Thermo Scientific, cat. no. EN0521) and cDNA was synthesized using the Applied Biosystems High Capacity cDNA Reverse Transcription Kit (Life Technologies, CA, USA; cat. no. 4368814). Amplicons of target components were generated by PCR reactions of 30-45 cycles of denaturation (95 °C, 20 s), annealing (58 °C, 30 s) and extension (68 °C, 30 s) with primers listed in **Table 2.** PCR products were separated on 1.5% agarose gels supplemented with 1 × RedSafe™ (iNtRON Biotechnology, Gyeonggi-do, Republic of Korea; cat. no. 21141) and visualized under UV light.

**Table 2: RT-PCR primers**

| **Target** | **Sequences** | **Amplicon size** | **SEQ ID NO:** | **Reference** |
|---|---|---|---|---|
| GAPD | 5'-ACATCGCTCAGACACCATG-3' | 143bp | 13 | Zhu X et al., J Cell Biochem 113,3069-85(2012) |
| H | 5'-TGTAGTTGAGGTCAATGAA-3' | | 14 | |
| GLUT1 | 5'-TGAACCTGCTGGCCTTC-3' | 399bp | 15 | Castro MA et al., Pflugers Arch 457, 519-28 (2008) |
| | 5'-GCAGCTTCTTTAGCACA-3' | | 16 | |
| GLUT2 | 5'-TCCAGCTACCGACAGCCTATTC-3' | 253bp | 17 | Limbert C et al., Cytotherapy 13, 802-13 (2011) |
| | 5'-AGATGGCACAAACAAACATCCC-3' | | 18 | |
| GLUT3 | 5'-AAGGATAACTATAATGG-3' | 411bp | 19 | Castro MA et al., Pflugers |
| | 5'-GGTCTCCTTAGCAGG-3' | | 20 | Arch 457, 519-28 (2008) |
| SGLT1 | 5'-TCCTGCTTGCTATTTTCTGGA-3' | 150bp | 21 | Kanwal A et al., Anal |
| | 5'-ATAATCGTGGGACAGTTGCTG-3' | | 22 | Biochem 429, 70-5 (2012) |
| SGLT2 | 5'-TCCTCACCCTCACGGTCTC-3' | 180bp | 23 | Kanwal A et al., Anal |
| | 5'-CTGGGGCTCATTCATCTCCA-3' | | 24 | Biochem 429, 70-5 (2012) |
| SUR1 | 5'-TCACACCGCTGTTCCTGCT-3' | 412bp | 25 | Du Q et al., Hum Reprod 25, |
| | 5'-AGAAGGAGCGAGGACTTGCC-3' | | 26 | 2774-82 (2010) |
| SUR2 | 5'-CATTGCCTACTTATTTCTCTCAG-3' | 474bp | 27 | Du Q et al., Hum Reprod 25, |
| | 5'-ACCATTCTGAAGAAAGCCAG-3' | | 28 | 2774-82 (2010) |
| Kir6.1 | 5'-CTGGCTGCTCTTCGCTATC-3' | 234bp | 29 | Du Q et al., Hum Reprod 25, |
| | 5'-AGAATCAAAACCGTGATGGC-3' | | 30 | 2774-82 (2010) |
| Kir6.2 | 5'-CCAAGAAAGGCAACTGCAACG-3' | 449bp | 31 | Du Q et al., Hum Reprod 25, |
| | 5'-ATGCTTGCTGAAGATGAGGGT-3' | | 32 | 2774-82 (2010) |

***Chemicals and Soft drinks.*** Ethanol (EtOH; cat. no. 02860), calcium chloride dihydrate (stock solution 0.5M in ddH₂O; cat. no. C7902), D-glucose (stock solution 1M in ddH₂O; cat. no. G-7021), D-mannitol (stock solution 0.1M in ddH₂O; cat. no. M4125), D-mannose (stock solution 1M in ddH₂O; cat. no. M6020), D-galactose (stock solution 0.1M in ddH₂O; cat. no. 48263), sucrose (stock solution 0.1M in ddH₂O; cat. no. S0389), D-maltose monohydrate (stock solution 0.1M in ddH₂O; cat. no. M9171), D-xylose (stock solution 0.1M in ddH₂O; cat. no. X1500), L-glutamine (stock solution 0.15M in ddH₂O; cat. no. G3126), 3-(N-Morpholino)propanesulfonic acid (MOPS; stock solution 0.1M in ddH₂O; cat. no. M1254), palmitic acid (stock solution 0.02M in EtOH; cat. no. P0500) and alloxan monohydrate (cat. no. A7413) were purchased from Sigma-Aldrich (Buchs, Switzerland). D-fructose (stock solution 0.1M in ddH₂O; cat. no. 161350010), L-leucine (stock solution 0.01M in glucose-free DMEM; cat. no. 125121000) and linoleic acid (stock solution 0.02M in EtOH; cat. no. 215040050) were purchased from Acros Organics (Geel, Belgium). L-glucose anhydrous (stock solution 1M in ddH₂O; cat. no. AB116919) was purchased from abcr GmbH (Karlsruhe, Germany). Potassium chloride (KCl; stock solution 4M in ddH₂O; cat. no. A3582) and sodium chloride (NaCl; stock solution 5M in ddH₂O; cat. no. A2942) were purchased from AppliChem (Darmstadt, Germany). Citric acid anhydrous (stock solution 0.1M in ddH₂O; cat. no. sc-211113) was purchased from Santa Cruz Biotechnology (Dallas, USA). Poly(L-lysine) hydrobromide (cat. no. PLKB50) was purchased from Alamanda Polymers (Alabama, USA). Trisodium citrate 2-hydrate (stock solution 0.1M in ddH₂O; cat. no. 6448) was purchased from Merck Millipore (Schaffhausen, Switzerland). Streptozotocin (cat. no. 1621) was purchased from Tocris Bioscience (Bristol, UK). Coke was purchased at local supermarkets, degassed by extensive shaking and directly administered to mice (4 × 200 µl).

***Animal experiments.*** The type 1 diabetes mouse model (T1D) was generated as described previously (Auslander D et al., Mol Cell 55, 397-408 (2014)). In brief, fasted mice (2x 18h/day) were injected with a single dose of freshly diluted alloxan monohydrate (ALX; 200 mg/kg in 300µl phosphate buffered saline) and persistent fasting hyperglycemia (>20mM) developed after 48h. The type 2 diabetes mouse model (T2D) was generated as described in (Arora S et al., Global J Pharmacol 3, 81-84 (2009)). In brief, fasted mice (20h/day) were injected with daily doses of freshly diluted streptozotocin (STZ; 40mg/kg in 250µl ice-cold sodium citrate buffer [pH 4.5, 0.01M, 0.11g/L NaCl]) for five consecutive days and chronic fasting hyperglycemia (>10mM) developed after 3 weeks. Glycemia of mice were measured with a commercial glucometer (Contour^{®} Next; Bayer HealthCare, Leverkusen, Germany; detection range: 0.5-35mM) purchased at local pharmacies. Intraperitoneal implants were produced by encapsulating transgenic HEK-293 cells into coherent alginate-poly-(L-lysine)-alginate beads (400µm; 500 cells/capsule) using an Inotech Encapsulator Research Unit IE-50R (EncapBioSystems Inc., Greifensee, Switzerland) set to the following parameters: a 200-µm nozzle with a vibration frequency of 1025Hz, a 25-mL syringe operated at a flow rate of 410 units and 1.12-kV voltage for bead dispersion (Ye H et al., PNAS 110, 141-146 (2013)). 5-9-weeks old female wild-type or ALX/STZ-pretreated CD-1 Swiss albino mice (Janvier Labs, Le Genest-Saint-Isle, France) were intraperitoneally injected with 1mL of glucose-free DMEM containing 1 x 10⁴ microcapsules. Blood serum was isolated using microtainer serum separating tubes (SST) according to the manufacturer's instructions (centrifugation for 5 min at 10 000 x g; Becton Dickinson, Plymouth, UK; cat. no. 365967). All experiments involving animals were performed according to the directive of the European Community Council (2010/63/EU), approved by the French Republic and carried out by Ghislaine Charpin-El Hamri (No. 69266309; project No. DR2013-01 (v2)) and Marie Daoud-El Baba (No. 69266310; project No. DR2013-01 (v2)) at the Institut Universitaire de Technologie, UCB Lyon 1, F-69622 Villeurbanne Cedex, France.

### Example 1: Coupling the β-cell-mimetic cascade of glycolysis-mediated calcium entry to a synthetic excitation-transcription coupling system

Glucose sensing was achieved by coupling the β-cell-mimetic cascade of glycolysis-mediated calcium entry to a synthetic excitation-transcription coupling system (D'Arco M and Dolphin AC, Sci Signal 5, pe34 (2012)) in human embryonic kidney cells. This human cell line is widely used in studies of ion channel activities (Thomas P and Smart TG, J Pharmacol Toxicol Methods 51, 187-200 (2005)) and shows optimal production capacities for antidiabetic proteins (Auslander D et al., Mol Cell 55, 397-408 (2014)). A cell-based assay was constructed in human embryonic kidney cells (HEK-293) to evaluate the stimulus strength of membrane depolarization with a quantitative reporter protein (Fig. 1A). When different calcium-responsive promoters (Hogan P et al., Genes Dev 17, 2205-2232 (2003)) were exposed to 75 mM potassium chloride (KCl), the synthetic promoter P_{NFAT-IL4}, which contains NFAT repeats derived from the murine interleukin 4 (IL4) promoter (Rooney JW et al., EMBO J 13, 625-633 (1994)), was most responsive to chemically induced membrane depolarization (Fig. 1B). Co-transfection of a voltage-gated calcium channel such as Caᵥ1.2 resulted in an amplified excitation-transcription coupling as well as a shift of the dose-response curve to higher sensitivity (Fig. 1C). The promoter architecture of five tandem NFAT_{IL4}-repeat sequences preceding a minimal eukaryotic TATA-box promoter ((NFAT_{IL4})₅-Pₘᵢₙ) showed optimal induction ratios between resting and depolarized states of membrane potentials (Fig. 1D). Because this promoter could also distinguish between signals generated by voltage-gated calcium channels of different activation thresholds (Fig. 1E), we used pMX57 ((NFAT_{IL4})₅-Pₘᵢₙ -SEAP-pA) as a reporter system for all subsequent studies that involved excitation-transcription coupling.

To experimentally evaluate the contributing effects of each β-cell-derived component for sensing glucose (GLUT2, GCK, K_{ATP}, Caᵥ1.3) with the pMX57-based depolarization-induced transcriptional system, a combinatorial screening approach (Fig. 2A) was used. The expression of K_{ATP} components did not show significant contributions to glucose sensing, whereas GLUT2 overexpression increased overall glucose-induced calcium-dependent transcription (Fig. 2A, condition #10), and GCK overexpression might cause toxic effects at higher extracellular glucose concentrations (Fig. 2A, conditions #6, #14, #16). Indeed, semi-quantitative transcriptional profiling confirmed that the GLUT1 and GLUT3 glucose transporters (Castro M et al., Pflugers Arch 457, 519-528 (2008); Elsner M et al., Diabetologia 45, 1542-1549 (2002)) as well as the K_{ATP} subunits of metabolism-dependent potassium channels are endogenously expressed in wild-type HEK-293 cells (Fig. 7A), and most mammalian cell types express at least one hexokinase isoform (Robey RB and Hay N, Oncogene 25, 4683-4696 (2006)). Therefore, ectopic expression of the Caᵥ1.3 channel was sufficient to confer glucose sensitivity to HEK-293 cells (Fig. 2A, inset), HeLa (Fig. 3A) and human mesenchymal stem cells (Fig. 3B). In contrast, putative glucose sensors reported in the literature, such as the T1R2/T1R3 sweet taste receptors (Jang H et al., PNAS 104, 15069-15074 (2007)), failed to mediate target gene regulation in response to glucose levels that are relevant for glycaemic control (Fig. 7B). It can be concluded that Caᵥ1.3 represents the missing link to reconstitute an intact, physiologically relevant glucose-sensing cascade in HEK-293 cells (Fig. 2B), and that only minimal, targeted engineering is required to mimic β-cell function in non-pancreatic human cells.

To quantitatively analyse the system, ensure consistency in the design steps, and eventually predict circuit operation *in vivo,* we developed a dynamic mathematical model for the β-cell-derived glucose-sensing cascade. Briefly, this ordinary differential equation (ODE) model covers the components shown in Fig. 2B, a detailed representation of the cell's electrophysiology and a previously developed, simplified representation of *in vivo* glucose-insulin interactions (Auslander D et al., Mol Cell 55, 397-408 (2014)). We parametrized the model with experimental data across conditions and experimental assays to establish a single, quantitative representation of the system. The model reproduced, among others, the potassium (Fig. 1E) and glucose (Fig. 2A) dose-response curves. We used the model for quantitative circuit characterization and, ultimately, to make essential predictions of *in vivo* behaviours.

### Example 2: Substrate specificity of the Caᵥ1.3/P_{NFAT-IL4}-constituted glucose-sensing system

To test the substrate specificity of the Caᵥ1.3/P_{NFAT-IL4}-constituted glucose-sensing system, Caᵥ1.3/pMX57-transgenic HEK-293 cells were cultured in cell culture medium containing different sugar compounds such as osmotic controls (Fig. 4A), common dietary sugars (Fig. 4B) and other nutrients (Fig. 7C). D-glucose and D-mannose were the only component among the tested substrates in HEK-293 cells that, at their physiologically relevant concentrations, activated SEAP expression from the synthetic excitation-transcription coupling system. HEK-293 cells use the GLUT1 transporter (Fig. 7A), which is most specific to glucose. Other mammalian cells express other transporters such as GLUT2 (not the case for HEK-293) which are also permeable for other carbohydrates such as D-fructose and D-galactose (Augustin R, IUBMB Life 62, 315-333 (2010)). Similar performances as with HEK-293 cells would be expected with other sugars if the sugar transport is mediated by e.g. GLUT2. Mannose is an epimer of glucose with an almost identical structure and may be transported into HEK-293 cells and metabolized by HEK-293 cells the same way as HEK-293 cells treats glucose. Capitalizing on the tight induction kinetics of the P_{NFAT3}-regulated gene expression system (Fig. 4C), and on the system's strict Caᵥ1.3-dependent activation (Fig. 4C and Fig. 7D), a stable human HEK-293_{NFAT-SEAP1} reporter cell line transgenic for P_{NFAT-IL4}-driven SEAP expression (Fig. 8) was constructed. Ectopic expression of the Caᵥ1.3 channel in HEK-293_{NFAT-SEAP1} resulted in improved induction ratios between low and high extracellular glucose concentrations (Fig. 5A; -4.6 fold-induction from 5mM to 25mM glucose) compared with its transiently constructed counterpart system (Fig. 2A; ∼2.6 fold-induction). The induction of Caᵥ1.3-transgenic HEK-293_{NFAT-SEAP1} cells was clearly significant with the first 24 h and reached maximal SEAP expression levels after culture in high-glucose medium for 48 h (Fig. 5B). Additionally, SEAP expression could be switched to dose-dependent regulation even after maintaining the Caᵥ1.3-transgenic HEK-293_{NFAT-SEAP1} cells in low-glucose conditions (2 mM) for different periods of time (Fig. 5C), and an independent time-course experiment showing glucose-mediated system sensitization as well as starvation-mediated desensitization confirmed the reversibility of the synthetic excitation-transcription coupling system (Fig. 5D). The mathematical model reproduced this behaviour quantitatively (Fig. 5, A, B and D; Fig. 9), and independent predictions for varying Caᵥ1.3 dosages agreed well with experiments (Fig. 5A), emphasizing the model's consistency across constructs and conditions.

### Example 3: Diabetes treatment with microencapsulated Cav1.3-transgenic HEK-293NFAT-SEAP1 cells

To test the application potential of the glucose-induced excitation-transcription coupling system for diabetes treatment, Caᵥ1.3-transgenic HEK-293_{NFAT-SEAP1} cells were microencapsulated into coherent, semi-permeable and immunoprotective alginate-poly-(L-lysine)-alginate beads and implanted them into the peritoneum of mice, where they become vascularized and connected to the animal's bloodstream with appropriate oxygen supply (Jacobs-Tulleneers-Thevissen D et al., Diabetologia 56, 1605-1614 (2013)). Also *in vivo,* the transcriptional regulation system operated in a dose- and Caᵥ1.3-dependent manner, as recapitulated by the same *in vitro* dynamic model coupled to a mathematical representation of mouse physiology (Fig. 10A). Importantly, the system translated the characteristic average fasting glycaemia values of wild-type as well as T1D- and T2D-diabetic mouse models into correspondingly expressed SEAP levels in the serum (Fig. 6A). When wild-type non-diabetic mice were fed concentrated sugar solutions, such as aqueous D-glucose (0.5 M) or Coca-Cola^{®} (11% w/w of total sugars), SEAP expression in the bloodstream was not significantly upregulated as compared with control groups receiving the same portions of water (Fig. 10B), thus indicating an intact glucose tolerance in healthy animals. Therefore, this glucose-induced transcriptional system might be tailored to exclusively target conditions of chronic hyperglycaemia while remaining insensitive to standard temporary glycaemic fluctuations such as nutrition.

State-of-the-art treatment options for diabetes mellitus are either long-acting drugs, such as stabilized GLP-1 variants, in which the frequency of drug injection can be reduced to weekly periods (T2D) (Trujillo J et al., Ther Adv Endocrinol Metab 6, 19-28 (2015)), or portable, external pump systems that self-sufficiently inject fast-acting insulin analogues according to the patient's instantaneous glycaemia (T1D) (Pickup JC, N Engl J Med 366, 1616-1624 (2012)). To test whether the expression levels achieved with the glucose-inducible excitation-transcription coupling system were compatible with antidiabetic therapeutic activities, HEK-293 cells were co-transfected with Caᵥ1.3 and the previously reported short human GLP-1 (shGLP1) construct (Ye H et al., Science 332, 1565-1568 (2011)) (pMX115; (NFAT_{IL4})₉-Pₘᵢₙ-shGLP1-pA) to engineer therapeutic mammalian cells that express GLP-1 exclusively under hyperglycaemic conditions (Fig. 10C). When implanting 5x10⁶ microencapsulated Caᵥ1.3/pMX115-transgenic HEK-293 cells into type-2 diabetic mice (Arora S et al., Global J Pharmacol 3, 81-84 (2009)), the self-sufficient exogenous expression of bioactive GLP-1 in the bloodstream (Fig. 6B) improved endogenous regulation of glucose-stimulated insulin secretion (Fig. 6C) and it substantially ameliorated glucose tolerance (Fig. 6D). Similarly, co-transfection of Caᵥ1.3 with an isogenic expression vector for insulin (mINS; modified rat insulin variant for optimized secretion) (Auslander D et al., Mol Cell 55, 397-408 (2014)) (pMX100; (NFAT_{IL4})₉-Pₘᵢₙ-mINS-pA) into HEK-293 cells generated glycaemia-triggered insulin-expressing cells (Fig. 10D). In agreement between experiments and model, self-sufficient insulin expression from Caᵥ1.3/pMX100-transgenic implants not only restored the typical insulin-deficiency (Polonsky KS, N Engl J Med 367, 1332-1340 (2012)) in a type-1 diabetic mouse model (Auslander D et al., Mol Cell 55, 397-408 (2014)) (Fig. 6E) but it also corrected the animals' persistent hyperglycaemia within 2-3 days (Fig. 6F). Importantly, hypoglycaemic side effects resulting from basal or excessive insulin expression at normoglycaemic levels that are often observed in classical insulin monotherapies (Pickup JC, N Engl J Med 366, 1616-1624 (2012)) were not detected (Fig. 6, E and F). Further development of the engineered system into the treatment option of choice to achieve lifelong glycaemic control in T1D patients also appears to be realistic because T1D mice developed hyperglycaemia with a high fatal outcome within one week (37.5% death cases without insulin treatment; n=24) whereas T1D animals treated with the Caᵥ1.3/pMX100-transgenic cell implant showed no hyperglycaemia and an improved life quality (0% death cases; n=24) (Fig. 11).

Implantation of microencapsulated mammalian cells into patients does not necessitate immunosuppression, as host and graft communicate via secretory metabolites that diffuse across a semi-permeable biocompatible membrane (Jacobs-Tulleneers-Thevissen D et al., Diabetologia 56, 1605-1614 (2013)). Since the first implantable alginate-poly-(L-lysine) capsules harbouring rat islet cells were presented almost 35 years ago, techniques for microencapsulated pancreatic β-cells have been continuously optimized for diabetes treatment, with several clinical trials already approved by governments. However, although current advances in stem cell research (Pagliuca FW et al., Cell 159, 428-439 (2014)) have successfully solved the previous issues of poor source availability and differentiation efficiency to generate adequate numbers of functional β-cells that elicit observable antidiabetic functions (Kobayashi N, Cell Transplant 15, 849-854 (2006)), regenerated pancreatic β-cells are generally restricted to the treatment of insulin-deficient type-1 diabetes (Bruin JE et al., Stem Cell Reports 4, 605-620 (2015)). Type-2 diabetes, however, is much more common, accounting for more than 95% of all diabetes cases and with a pathogenesis that often involves an impaired sensitivity of body cells to excessive levels of circulating insulin (Johnson AM and Olefsky JM, Cell 152, 673-684 (2013)). Glucagon-like peptide 1 (GLP-1) is an incretin hormone naturally released from the intestine after meal ingestion that not only acts on β-cells to stimulate their postprandial release of insulin (Drucker DJ et al., Lancet 368, 1696-1705 (2006)) but also circulates to other somatic cells to (i) promote satiety, (ii) improve hepatic insulin sensitivity, (iii) slow gastric emptying and (iv) inhibit glucagon secretion (Ye H et al., PNAS 110, 141-146 (2013)). Currently, subcutaneous injection of long-acting GLP-1 analogues is the state-of-the-art treatment option for most type-2 diabetes cases (Trujillo J et al., Ther Adv Endocrinol Metab 6, 19-28 (2015)).

The quest for a cell-based glucose sensor has always been in great demand for biomedical research (Auslander D et al., Mol Cell 55, 397-408 (2014)). Although a variety of putative glucose-sensing components, including GPCRs (Jang H et al., PNAS 104, 15069-15074 (2007)), bacterial transcriptional repressors (Gaigalat L et al., BMC Mol Biol 8, 104 (2007)) and human nuclear receptors (Mitro N et al., Nature 445, 219-223 (2007)) have been characterized, to the best of our knowledge, a successful translation of the glucose input into a transcriptional message that includes antidiabetic activities *in vivo* has not yet been achieved. In this work, we used the low threshold voltage-gated calcium channel Caᵥ1.3, which permits long-lasting Ca²⁺ influxes during weak membrane depolarizations (Lipscombe D et al., J Neurophysiol 92, 2633-2641 (2004)), to sense glycaemia-relevant extracellular glucose levels but to remain insensitive to other potential trigger compounds such as metabolites and salts within their physiologically relevant concentration range (Fig. 4B, Fig. 7C); it represented the single missing component for engineering a glucose-inducible transcription unit in human embryonic kidney cells. Therefore, this work indicated that the glucose-sensing mechanism of glycolysis-stimulated calcium entry may not be restricted to β-cells and that it might serve as a blueprint for conferring glucose responsiveness to other mammalian cell types. Collectively, our engineered glucose-inducible excitation-transcription system integrated all potential advantages of an improved alternative for diabetes therapy in future clinical applications: First, unlike technology profiling glucose levels indirectly via low blood pH during diabetic ketoacidosis (Auslander D et al., Mol Cell 55, 397-408 (2014)), our cell-based glucose sensor directly quantifies hyperglycaemia in the absence of any interference with other types of acidosis such as lactic or alcohol acidosis. Second, hyperglycaemia-inducible expression of a glycaemia-lowering protein, such as insulin, allows for the engineering of a closed-loop prosthetic gene circuit that self-(in)activates in an automated manner, i.e., insulin expression occurs exclusively under diabetic hyperglycaemic conditions and remains inactive under normoglycaemic conditions. Such a self-sufficient theranostic device (Kojima R et al., Curr Opin Chem Biol 28, 29-38 (2015)) would guarantee delivery of a therapeutic protein at optimal bioavailable concentrations. Third, the device enables flexible and patient-centered customization of the output protein. The hyperglycaemic disease marker can be coupled to the self-sufficient expression of not only insulinogenic agents such as insulin (T1D) and GLP-1 (T2D) but in principle to any drug against other hyperglycaemia-related diseases, such as cardiovascular disease or the metabolic syndrome. Fourth, engineering of synthetic biology-inspired gene circuits is economical in cost and time when compared to other techniques such as islet transplantation or stem cell differentiation, thus compensating for the main limitation of a regular need for cell replacements in microcapsule implants (Yang HK and Yoon KH, J Diabetes Complications 5, 737-43 (2015)).

Although an ideal implant genotype for T1D therapy might also include postprandial insulin release to attenuate immediate perturbations of blood glucose levels following dietary sugar intake, our experimental and computational analyses showed that the glucose-induced insulin expression system achieved rapid attenuation of life-threatening hyperglycaemia in a T1D animal model by restoring near-homeostatic blood insulin and glucose levels in a self-sufficient and (predicted) robust manner. In particular, glycaemic control in T1D mice was restored in less than one week following implantation - a response time that compares favourably with experimental cell-based therapies in animals using pancreatic progenitor cells (30 weeks; (Rezania A et al., Diabetes 61, 2016-2029 (2012)) or β-cell mimetics produced from human pluripotent stem cells by a seven-stage *in vitro* differentiation protocol (40 days; (Rezania A et al., Nat Biotechnol 32, 1121-1133 (2014)). Furthermore, the animal experiments demonstrated an absolutely increased survival rate already with the current implant version and within only one week of treatment, which should be the foremost criterion to judge the system's efficiency. This work is therefore considered as a proof-of-principle study that introduces an attractive alternative concept for diabetes treatment with therapeutic features that have already been achieved at a standard of practical relevance.

## Claims

1. A recombinant cell comprising
a nucleic acid construct comprising a promoter which is responsive to carbohydrate metabolism of said recombinant cell, wherein the promoter is operably linked to a gene coding for a therapeutic protein.

2. The recombinant cell of claim 1, wherein the recombinant cell further comprises a nucleic acid construct coding for a cellular component for sensing extracellular carbohydrates.

3. The recombinant cell of claim 2, wherein the cellular component for sensing extracellular carbohydrates is a membrane protein or a fragment thereof or a subunit of a membrane protein or a fragment thereof.

4. The recombinant cell of claim 2, wherein the cellular component for sensing extracellular carbohydrates is a membrane protein or a fragment thereof or a subunit of a membrane protein or a fragment thereof, wherein the membrane protein is selected from the group consisting of G-protein coupled receptors, SLC2A family glucose transporters, SLC5A family sodium-glucose linked transporters, potassium channels, calcium channels and sodium channels.

5. The recombinant cell of claim 2, wherein the cellular component for sensing extracellular carbohydrates is a voltage-gated calcium channel.

6. The recombinant cell of anyone of claims 1-5, wherein the promoter which is responsive to carbohydrate metabolism is responsive to a physiological effect of membrane depolarization caused by the carbohydrate metabolism of said recombinant cell.

7. The recombinant cell of anyone of claims 1-5, wherein the promoter which is responsive to carbohydrate metabolism is a calcium-responsive promoter.

8. The recombinant cell of anyone of claims 1-5, wherein the promoter which is responsive to carbohydrate metabolism is a calcium-responsive promoter comprising nucleic acid sequences bound by transcription factors of the NFAT family, the NFkB family, the AP-1 family, and/or the CREB family and/or cFOS.

9. The recombinant cell of anyone of claims 1-8, wherein the therapeutic protein is an insulinogenic agent selected from the group consisting of GLP1R-agonists, insulin, insulin analogues, growth hormones, insulin-like growth factors; an anorexic hormone; or a protein that activates brown fat metabolism.

10. The recombinant cell of anyone of claims 1-9, wherein the therapeutic protein is an agent against a metabolic disease, wherein the metabolic disease is selected from the group consisting of T1D (type-1 diabetes), T2D (type-2 diabetes), diabetic ketoacidosis, obesity, cardiovascular disease, the metabolic syndrome and cancer.

11. An encapsulated cell comprising the recombinant cell of anyone of claims 1-10 and a semi-permeable membrane.

12. The recombinant cell of anyone of claims 1-10 or the encapsulated cell of claim 11 for use as a medicament.

13. The recombinant cell of anyone of claims 1-10 or the encapsulated cell of claim 1 for use in a method for the prevention, delay of progression or treatment of a metabolic disease in a subject.

14. A method of producing a recombinant cell expressing a therapeutic protein, said method comprising the steps of:
(a) obtaining a population of cells;
(b) transfecting said population of cells with a nucleic acid construct comprising a promoter which is responsive to a product of the carbohydrate metabolism of said cell, wherein the promoter is operably linked to a gene coding for a therapeutic protein;
(c) incubating the population of transfected cell in the presence of carbohydrates for a sufficient time to permit the transfected cells to express a therapeutic protein.

15. A method to deliver a nucleic acid construct to a cell, wherein the nucleic acid construct comprises a promoter which is responsive to carbohydrate metabolism of said cell, wherein the promoter is operably linked to a gene coding for a therapeutic protein, comprising administering said nucleic acid construct to said cell, whereby said gene coding for a therapeutic protein is expressed in said cell in response to carbohydrate stimulation.


SEQUENCE LISTING

<110>  ETH Zurich

<120>  B-CELL-MIMETIC CELLS

<130>  2015-165

<160>  39

<170>  PatentIn version 3.5

<210>  1
<211>  114
<212>  DNA
<213>  Artificial Sequence

<220>
<223>  OMX59 primer

<400>  1
cgcgtgctag cagcctgacg tttcagagac tgacgtttca gagactgacg tttcagagac      60

tgacgtttca gatctctcga ggtcgacagc ggagactcta gagggtatat aatg           114


<210>  2
<211>  30
<212>  DNA
<213>  Artificial Sequence

<220>
<223>  OMX24 primer

<400>  2
cttgagcaca tagcctggac cgtttccgta                                       30


<210>  3
<211>  116
<212>  DNA
<213>  Artificial Sequence

<220>
<223>  OMX63 primer

<400>  3
cgcgtgctag ctacattgga aaattttata cacgttctag ctacattgga aaattttata      60

cacgttctag ctacattgga aaattttata cacgttagac tctagagggt atataa         116


<210>  4
<211>  233
<212>  DNA
<213>  Artificial Sequence

<220>
<223>  PNFAT_IL4x5

<400>  4
cgcgtgctag ctacattgga aaattttata cacgttctag ctacattgga aaattttata      60

cacgttctag ctacattgga aaattttata cacgttctag ctacattgga aaattttata 120

cacgttctag ctacattgga aaattttata cacgttagac tctagagggt atataatgga 180

agctcgactt ccagcttggc aatccggtac tgttggtaaa gaattcgccc acc 233

<210> 5
<211> 293
<212> DNA
<213> Artificial Sequence

<220>
<223> PNFAT_IL4x7

<400> 5
cgcgtgctag ctacattgga aaattttata cacgttctag ctacattgga aaattttata 60

cacgttctag ctacattgga aaattttata cacgttctag ctacattgga aaattttata 120

cacgttctag ctacattgga aaattttata cacgttctag ctacattgga aaattttata 180

cacgttctag ctacattgga aaattttata cacgttagac tctagagggt atataatgga 240

agctcgactt ccagcttggc aatccggtac tgttggtaaa gaattcgccc acc 293

<210> 6
<211> 353
<212> DNA
<213> Artificial Sequence

<220>
<223> PNFAT_IL4x9

<400> 6
cgcgtgctag ctacattgga aaattttata cacgttctag ctacattgga aaattttata 60

cacgttctag ctacattgga aaattttata cacgttctag ctacattgga aaattttata 120

cacgttctag ctacattgga aaattttata cacgttctag ctacattgga aaattttata 180

cacgttctag ctacattgga aaattttata cacgttctag ctacattgga aaattttata 240

cacgttctag ctacattgga aaattttata cacgttagac tctagagggt atataatgga 300

agctcgactt ccagcttggc aatccggtac tgttggtaaa gaattcgccc acc 353

<210> 7
<211> 49
<212> DNA
<213> Artificial Sequence

<220>
<223> OMX70 primer

<400> 7
ctgttggtaa agaattcgcc caccatgaag atcatcctgt ggctgtgtg 49

<210> 8
<211> 46

<212>  DNA
<213>  Artificial Sequence

<220>
<223>  OMX71 primer

<400>  8
ggagtcgacg cgtgaagcgg ccggcctcat ttaccaggag agtggg                    46


<210>  9
<211>  49
<212>  DNA
<213>  Artificial Sequence

<220>
<223>  OMX72 primer

<400>  9
ctgttggtaa agaattcgcc caccatggcc ctgtggatgc gcttcctgc                 49


<210>  10
<211>  31
<212>  DNA
<213>  Artificial Sequence

<220>
<223>  OMX73 primer

<400>  10
ctgaaacata aaatgaatgc aattgttgtt g                                    31


<210>  11
<211>  34
<212>  DNA
<213>  Artificial Sequence

<220>
<223>  OMX100 primer

<400>  11
ctggaattcc accatgctgg acgacagagc cagg                                 34


<210>  12
<211>  44
<212>  DNA
<213>  Artificial Sequence

<220>
<223>  OMX101 primer

<400>  12
ctctagatgc atgctcgagt cactggccca gcatacaggc cttc                      44


<210>  13
<211>  19
<212>  DNA
<213>  Artificial Sequence

<220>
<223>  GAPDH forward primer

<400>  13
acatcgctca gacaccatg                                                    19


<210>  14
<211>  19
<212>  DNA
<213>  Artificial Sequence

<220>
<223>  GAPDH reverse primer

<400>  14
tgtagttgag gtcaatgaa                                                    19


<210>  15
<211>  17
<212>  DNA
<213>  Artificial Sequence

<220>
<223>  GLUT1 forward primer

<400>  15
tgaacctgct ggccttc                                                      17


<210>  16
<211>  17
<212>  DNA
<213>  Artificial Sequence

<220>
<223>  GLUT1 reverse primer

<400>  16
gcagcttctt tagcaca                                                      17


<210>  17
<211>  22
<212>  DNA
<213>  Artificial Sequence

<220>
<223>  GLUT2 forward primer

<400>  17
tccagctacc gacagcctat tc                                                22


<210>  18
<211>  22
<212>  DNA
<213>  Artificial Sequence

<220>
<223>  GLUT2 reverse primer

<400>  18
agatggcaca aacaaacatc cc                                                22

<210>  19
<211>  17
<212>  DNA
<213>  Artificial Sequence

<220>
<223>  GLUT3 forward primer

<400>  19
aaggataact ataatgg                                                      17

<210>  20
<211>  15
<212>  DNA
<213>  Artificial Sequence

<220>
<223>  GLUT3 reverse primer

<400>  20
ggtctcctta gcagg                                                        15

<210>  21
<211>  21
<212>  DNA
<213>  Artificial Sequence

<220>
<223>  SGLT1 forward primer

<400>  21
tcctgcttgc tattttctgg a                                                 21

<210>  22
<211>  21
<212>  DNA
<213>  Artificial Sequence

<220>
<223>  SGLT1 reverse primer

<400>  22
ataatcgtgg gacagttgct g                                                 21

<210>  23
<211>  19
<212>  DNA
<213>  Artificial Sequence

<220>
<223>  SGLT2 forward primer

<400>  23
tcctcaccct cacggtctc                                                    19

<210>  24
<211>  20
<212>  DNA
<213>  Artificial Sequence

<220>
<223>  SGLT2 reverse primer

<400>  24
ctggggctca ttcatctcca                                                    20


<210>  25
<211>  19
<212>  DNA
<213>  Artificial Sequence

<220>
<223>  SUR1 forward primer

<400>  25
tcacaccgct gttcctgct                                                     19


<210>  26
<211>  20
<212>  DNA
<213>  Artificial Sequence

<220>
<223>  SUR1 reverse primer

<400>  26
agaaggagcg aggacttgcc                                                    20


<210>  27
<211>  23
<212>  DNA
<213>  Artificial Sequence

<220>
<223>  SUR2 forward primer

<400>  27
cattgcctac ttatttctct cag                                                23


<210>  28
<211>  20
<212>  DNA
<213>  Artificial Sequence

<220>
<223>  SUR2 reverse primer

<400>  28
accattctga agaaagccag                                                    20


<210>  29
<211>  19

<212>  DNA
<213>  Artificial Sequence

<220>
<223>  Kir6.1 forward primer

<400>  29
ctggctgctc ttcgctatc                                                    19


<210>  30
<211>  20
<212>  DNA
<213>  Artificial Sequence

<220>
<223>  Kir6.1 reverse primer

<400>  30
agaatcaaaa ccgtgatggc                                                   20


<210>  31
<211>  21
<212>  DNA
<213>  Artificial Sequence

<220>
<223>  Kir6.2 forward primer

<400>  31
ccaagaaagg caactgcaac g                                                 21


<210>  32
<211>  21
<212>  DNA
<213>  Artificial Sequence

<220>
<223>  Kir6.2 reverse primer

<400>  32
atgcttgctg aagatgaggg t                                                 21


<210>  33
<211>  32
<212>  DNA
<213>  Artificial Sequence

<220>
<223>  Pmin

<400>  33
tagagggtat ataatggaag ctcgacttcc ag                                     32


<210>  34
<211>  30
<212>  DNA
<213>  Artificial Sequence

<220>
<223>  NFAT binding site of murine interleukin (IL)-4 promoter

<400>  34
ctagctacat tggaaaattt tatacacgtt                                        30


<210>  35
<211>  255
<212>  PRT
<213>  Artificial Sequence

<220>
<223>  shGLP1

<400>  35

His Gly Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly Arg
            20                  25                  30

Ser Gly Cys Lys Pro Cys Ile Cys Thr Val Pro Glu Val Ser Ser Val
        35                  40                  45

Phe Ile Phe Pro Pro Lys Pro Lys Asp Val Leu Thr Ile Thr Leu Thr
    50                  55                  60

Pro Lys Val Thr Cys Val Val Val Asp Ile Ser Lys Asp Asp Pro Glu
65                  70                  75                  80

Val Gln Phe Ser Trp Phe Val Asp Asp Val Glu Val His Thr Ala Gln
                85                  90                  95

Thr Gln Pro Arg Glu Glu Gln Phe Asn Ser Thr Phe Arg Ser Val Ser
            100                 105                 110

Glu Leu Pro Ile Met His Gln Asp Trp Leu Asn Gly Lys Glu Phe Lys
        115                 120                 125

Cys Arg Val Asn Ser Ala Ala Phe Pro Ala Pro Ile Glu Lys Thr Ile
    130                 135                 140

Ser Lys Thr Lys Gly Arg Pro Lys Ala Pro Gln Val Tyr Thr Ile Pro
145                 150                 155                 160

Pro Pro Lys Glu Gln Met Ala Lys Asp Lys Val Ser Leu Thr Cys Met
                165                 170                 175

Ile Thr Asp Phe Phe Pro Glu Asp Ile Thr Val Glu Trp Gln Trp Asn
            180                 185                 190

Gly Gln Pro Ala Glu Asn Tyr Lys Asn Thr Gln Pro Ile Met Asp Thr
        195                 200                 205

Asp Gly Ser Tyr Phe Val Tyr Ser Lys Leu Asn Val Gln Lys Ser Asn
    210                 215                 220

Trp Glu Ala Gly Asn Thr Phe Thr Cys Ser Val Leu His Glu Gly Leu
225                 230                 235                 240

His Asn His His Thr Glu Lys Ser Leu Ser His Ser Pro Gly Lys
                245                 250                 255

<210>  36
<211>  110
<212>  PRT
<213>  Artificial Sequence

<220>
<223>  mINS

<400>  36

Met Ala Leu Trp Met Arg Phe Leu Pro Leu Leu Ala Leu Leu Val Leu
1               5                   10                  15

Trp Glu Pro Lys Pro Ala Gln Ala Phe Val Lys Gln His Leu Cys Gly
            20                  25                  30

Pro His Leu Val Glu Ala Leu Tyr Leu Val Cys Gly Glu Arg Gly Phe
        35                  40                  45

Phe Tyr Thr Pro Lys Ser Arg Arg Lys Arg Glu Asp Pro Gln Val Pro
    50                  55                  60

Gln Leu Glu Leu Gly Gly Gly Pro Glu Ala Gly Asp Leu Gln Thr Leu
65                  70                  75                  80

Ala Leu Glu Val Ala Arg Gln Lys Arg Gly Ile Val Asp Gln Cys Cys
                85                  90                  95

Thr Ser Ile Cys Ser Leu Tyr Gln Leu Glu Asn Tyr Cys Asn
            100                 105                 110

<210>  37
<211>  60
<212>  DNA
<213>  Artificial Sequence

<220>
<223>  OFS200 Primer

<400>  37
tgttggtaaa gaattcgccc accaagcttt aagccaccat ggagagcgac gagagcggcc        60

<210>  38
<211>  25
<212>  DNA
<213>  Artificial Sequence

<220>
<223>  OMX78 Primer

<400>  38
ctttaaaaaa cctcccacac ctccc                                              25

<210>  39
<211>  186
<212>  DNA
<213>  Artificial Sequence

<220>
<223>  PNFAT_IL4x3

<400>  39
tggccggtac ctgagctcgc tagctacatt ggaaaatttt atacacgttc tagctacatt        60

ggaaaatttt atacacgttc tagctacatt ggaaaatttt atacacgtta gactctagag       120

ggtatataat ggaagctcga cttccagctt ggcaatccgg tactgttggt aaagaattcg       180

cccacc                                                                  186